# EUROPÄISCHE PATENTSCHRIFT

(11) **EP 2 773 612 B1**
(45) Veröffentlichungstag und Bekanntmachung des Hinweises auf die Patenterteilung: **12.12.2018**
(21) Anmeldenummer: 12775712.8
(22) Anmeldetag: 26.10.2012
(51) Int. Cl.: C07C 263/18, C08G 18/72

(54) **VERFAHREN ZUR HERSTELLUNG VON IN LÖSUNGSMITTELN FLOCKULATIONSSTABILEN POLYISOCYANATEN VON (CYCLO)ALIPHATISCHEN DIISOCYANATEN**
METHOD FOR PRODUCING POLYISOCYANATES, WHICH ARE FLOCCULATION-STABLE IN SOLVENTS, FROM (CYCLO)ALIPHATIC DIISOCYANATES
PROCÉDÉ DE PRODUCTION DE POLYISOCYANATES DE DIISOCYANATES (CYCLO)ALIPHATIQUES RÉSISTANTS À LA FLOCULATION DANS LES SOLVANTS

(30) Priorität: 28.10.2011 EP 11187010
(43) Veröffentlichungstag der Anmeldung: 10.09.2014
(73) Patentinhaber: BASF SE, 67056 Ludwigshafen am Rhein (DE)
(72) Erfinder: SCHÄFER, Harald, 68219 Mannheim (DE); GOMYO, Shintaro, Nishinomiya, Hyogo, 6638006 (JP); HE, Jing, Shangai, 200137 (CN); BINDER, Horst, 68623 Lampertheim (DE); HAN, Liang, Shangai, 200231 (CN); LEE, Woosuk, Pyeong-geo dong, Jinjoo city (KR)
(74) Vertreter: BASF IP Association
(86) Internationale Anmeldenummer: PCT/EP2012/071201
(87) Internationale Veröffentlichungsnummer: WO 2013/060809

(56) Entgegenhaltungen:
- EP-A1- 0 341 516
- WO-A1-2008/068197
- DE-A1- 19 824 490
- US-B1- 6 291 577

## Beschreibung

Die vorliegende Erfindung betrifft ein neues Verfahren zur Herstellung von in Lösungsmitteln flockulationsstabilen Polyisocyanaten von (cyclo)aliphatischen Diisocyanaten.

para-Toluolsulfonylisocyanat (H₃C(C₆H₄)SO₂-NCO) und Triethylorthoformat sind literaturbekannte Beispiele für Wasserfänger, die in Polyisocyanaten zur Stabilisierung eingesetzt werden. Diese Stabilisierung umfasst u.a. die Verhinderung der Flockulation aliphatischer Polyisocyanate in verdünnter Form in Lösungsmitteln wie sie für Lackanwendungen eingesetzt werden.
Es wird dabei davon ausgegangen, dass Wasser fangende Verbindungen im Wettbewerb zum Polyisocyanat verhindern, dass Isocyanat-Gruppen mit Wasser zu Aminen hydrolysieren und diese mit weiterem Polyisocyanat zu hochfunktionellen (Poly)-Harnstoffpolyisocyanaten reagieren. Diese weisen eine schlechte Löslichkeit in Polyisocyanaten und deren Lösungen auf und können dabei Flocken und Niederschläge bilden.

Unter Flockulation im Sinne dieses Patents wird umfassend eine mit dem bloßen Auge erkennbare Feststoffbildung verstanden. Dies geht von einer leichten Trübung, feinen, erst durch rotierende Bewegung um die Längsachse des Lagergefäßes aufwirbelnden Niederschlägen, bis zur Bildung schwebender Flocken und schwerer Niederschläge.

Nachteilig an Wasserfängern ist, dass diese (mindestens) stöchiometrisch zur Menge des vorhandenen oder zu erwartenden Wasser zugegeben werden müssen. Bei para-Toluolsulfonylisocyanat entspricht dies der 12-fachen, bei Triethylorthoformat der 9-fachen Gewichtsmenge an Wasser. Herstellerseitig wird beim para-Toluolsulfonylisocyanat sogar die doppelte Äquivalentmenge gegenüber Wasser empfohlen, respektive Mengen an para-Toluolsulfonylisocyanat von 0,5-4,0 % und 1-3 % Triethylorthoformat auf das Gesamtgewicht der Formulierung. Werden solche Mengen eingesetzt, reduzieren sie allein durch den Verdünnungsfaktor entsprechend die NCO-Gruppen, was nachteilig für die Lackeigenschaften ist.

Para-toluolsulfonylisocyanat ist ein hochreaktives monofunktionelles Isocyanat welches statt des zu schützenden Polyisocyanats bevorzugt mit Wasser reagiert. Dabei bildet sich inertes Sulfonamid und wie bei der Reaktion des Polyisocyanats selber Kohlendioxid, gegebenenfalls unter Aufbau von Druck im Lagergefäß.

DE 124,590 beschreibt die Anwendung von Sulfonylisocyanaten als wasserbindende Komponenten in Polyurethanpräpolymeren. EP 86871 beschreibt Nachteile dieser Verbindungen, da das durch Reaktion mit Wasser entstehende Tosylamid wegen seiner ausgeprägten Kristatallisationstendenz zur Stippenbildung im Lack führt. Zudem ist das Tosylisocyanat so hochreaktiv, dass es mit Wasser außerordentlich heftig reagiert.

JP 2001/081,400 beschreibt feuchtigkeitshärtende Urethanharz-Lackzusammensetzungen, enthaltend ein Urethanpräpolymer mit zwei oder mehr Isocyanatgruppen in einem Molekül, z.B. aus dem aromatischen Diphenylmethandiisocyanat (MDI) und Polypropylenglykol, blockierte Amine, zwei Arten von Wasserfängern und einen Entschäumer. Wasserfänger sind z.B. eine Kombination aus para-Toluolsulfonylisocyanat und Triethylorthoformat. Hintergrund ist eine Lagerstabilität zur Vermeidung einer vorzeitigen Hydrolyse des blockierten Amins und Aushärtung der latenten Reaktionsmischung.

US 6,291,577 offenbart eine Methode Feuchtigkeit in Polyisocyanatformulierungen einzufangen, indem man der Polyisocyanatformulierung Wasserfänger zumischt enthaltend a) Ditert.-butylhydroxytoluol ausgewählt aus der Gruppe von 2,6-Ditert.-butyl-hydroxytoluol und 2-tert.-Butyl-hydroxytoluol und b) Alkylestern der Toluolsulfonsäure mit mindestes 90% Anteil an para-Alkylester, optional in wenigstens einem Lösungsmittel. Beispielsweise genannt sind ein Paket an Feuchtigkeitsfängern bestehend aus 0,19 % Bis-(tert.-butyl)-hydroxytoluol (BHT) und 1,0 % para-Toluolsulfonsäure-methylester, gegebenenfalls in Kombination mit anderen Feuchtigkeitsfängern, bezogen auf ein Isocyanuratgruppen aufweisendes Polyisocyanat auf Basis von Hexamethylendiisocyanat als 40 %-ige Lösung in n-Butylacetat. Entsprechende Mischungen sind 11 Wochen lagerstabil, ohne dass es zu Trübung oder Vergilbung kommt. Nachteilig an dem Verfahren ist, dass die Mengen an Feuchtigkeitsfänger sehr hoch sind, so dass der NCO-Wert des Polyisocyanats durch das Feuchtigkeitsfängerpaket durch Verdünnung um 1% reduziert wird. Zudem sind nur der Methyl- und Ethylester der para-Toluolsulfonsäure kommerziell gut zugänglich. Zudem ist die Löslichkeiten von para-Toluolsulfonsäuremethylester schlecht. Sie löst sich z.B. bei Raumtemperatur zwar 1 % in Butylacetat, aber nicht 10%-ig.

US 5,328,635 offenbart Iminoalkohole und Oxazolidine als Wasser- und Formaldehydfänger mit diversen Vorteilen, so als Korrosionsinhibitor, Reaktivverdünner, Rheologiemodifizierer und gegen Schaumbildung. Eine Eignung als Antiflockulanz für die Lagerung von Polyisocyanaten in Lösungsmitteln wird nicht beschrieben. Nachteilig für eine Anwendung im Sinne der Erfindung wäre, dass solche Verbindungen als Wasserfänger stöchiometrisch zugegeben werden müssen, und Auswirkungen auf die Eigenschaften der Polyisocyanate in Lackformulierungen haben, so dass sich stabilisierte und unstabilisierte Polyisocyanate in Lackformulierungen unterschiedlich verhalten und somit in der Anwendung Probleme bereiten. Ebenfalls nachteilig ist, dass Amin-haltige Verbindungen wie Iminoalkohole und Oxazolidine abhängig von den Lagerbedingungen zur Gelbfärbung neigen und einen Einfluss auf die Reaktivität des Lacksystems haben, z.B. die Verarbeitungszeit verkürzen. Oxazolidine reagieren mit Wasser unter Ringöffnungen zu difunktionellen Isocyanat-reaktiven Aminoalkoholen. Diese oligomerisieren mit Polyisocyanat. Dies kann zu einem Viskositätsanstieg bei Lagerung kommen.

EP 86,971 beschreibt den Einsatz von Isocyanatosilanen zur Verbesserung der Lagerstabilität von Polyisocyanaten in Bezug auf Viskosität. Es gibt keinen Hinweis auf Stabilisierung von Polyisocyanat-(Lösungen) gegenüber Flockulation. Isocyanatosilane stehen kommerziell nur sehr eingeschränkt zur Verfügung.

US 2008/0257214 beschreibt den Einsatz bestimmter Trimethylsilyl-Gruppen-haltiger Verbindungen wie Bistrimethylsilylacetamid oder Hexamethyldisilazan als Wasserfänger zum Verhindern von Trübungen und Kohlenstoffdioxidbildung von Polyisocyanaten in Lösungsmitteln. Wie bei den meisten Trockenmitteln müssen diese Verbindungen mindestens stöchiometrisch zugegeben werden. Die entstehenden Spaltprodukte verbrauchen NCO-Gruppen.

EP 203,874 offenbart Trialkyl-chloro-zinnverbindungen zur Stabilisierung von Polyisocyanaten in organischen Lösungsmitteln gegen Flockulat. Triorgano-Zinnverbindungen sind hochgradig toxisch.

WO 08 /116607 beschreibt Zweikomponenten-Lacksysteme, umfassend als Komponenten
a) ein oder mehrere Polyisocyanate,
b) eine oder mehrere mit Komponente (a) reaktive, oligomere und/oder polymere Verbindungen und
c) ein oder mehrere Phosphonate ausgewählt aus der Gruppe der Phosphonsäurediester und Diphosphonsäurediester
wobei
(i) Komponente (b) alle im Zweikomponenten-Lacksystem enthaltenen mit Komponente (a) reaktiven, oligomeren und/oder polymeren Verbindungen enthält und
(ii) Komponente (b) höchstens 15 Gew. % bezogen auf das Gesamtgewicht der Komponente (b) an oligomeren und/oder polymeren Verbindungen enthält, die gegenüber Isocyanat reaktive Aminogruppen enthält.

Das System hat den Vorteil, dass es eine gute Topfzeit und gleichzeitig ein gutes Erscheinungsbild und ausreichende Härte gewährleistet.
In den Beispielen der Patentanmeldung werden die Komponenten (b), (c), Lösungsmittel und Additive vor der Anwendung direkt vermischt. Es gibt keinen Hinweis auf eine antiflockulierende Wirkung der Komponenten (c).

DE 19,908,793 beschreibt ein Verfahren zur Herstellung eines Aktivisocyanats in Anwesenheit einer Dialkylphosphonsäure (gleich Dialkylphosphonsäureester), bei dem insbesondere bei der Herstellung keine Verfestigung oder Eintrübung erfolgt und ein lagerstabiles Aktivisocyanat erhalten wird. Nach den erfindungsgemäßen Beispielen und Seite 2, Zeile 57 handelt es sich bei dem Aktivisocyanat um aromatische Carbodiimide und Uretonimine. Diese werden nach ihrer Herstellung in Substanz gelagert.
Es findet sich kein Hinweis auf eine stabilisierende Wirkung auf andere Polyisocyanate als Carbodiimide und Uretonimine, insbesondere nicht auf eine antiflockulierende Wirkung.

WO 2008 / 116895 beansprucht Polyisocyanat-Zusammensetzungen enthaltend (a) Polyisocyanat, (b) Urethanisierungskatalysator, (c) Phosphonat und gegebenenfalls (d) sterisch gehindertes Phenol, (e) Lösungsmittel, (f) saure Stabilisatoren, (g) weitere lacktypische Additive. Diese Polyisocyanat-Zusammensetzungen in Anwesenheit eines Urethanisierungskatalysator sind bei Lagerung farbstabil. Die Patentanmeldung enthält keinen Hinweis auf Flockulationsstabilität von Polyisocyanaten in hoher Verdünnung in Lösungsmitteln, insbesondere nicht in Abwesenheit von Lewis-Säuren.

EP 341,516 offenbart Polyisocyanate enthaltend (ggf. Methyl-substitutierte) 2-, und 3-Chlorpropionsäure. Als Polyisocyanate werden Polyesterurethane aus Hydroxypolyestern und dem aromatischen Toluylendiisocyanat beschrieben. Die Säuren weisen eine stabilisierende Wirkung in Bezug auf Verfärbung während Lagerung (in Essigester als Lösungsmittel) sowie der Reaktivität der Polyesterurethane gegenüber Ketiminen nach der Lagerung auf. Der Einsatz in aliphatischen Polyisocyanaten wird nicht beschrieben. Lagerstabilität in Bezug auf Flockulation in Feuchtigkeit enthaltenden Lösungsmitteln wird nicht beschrieben.

WO 2008/68197 offenbart Polyisocyanate, enthaltend insbesondere Methoxyessigsäure als Stabilisator. Lagerstabilität in Bezug auf Flockulation in Feuchtigkeit enthaltenden Lösungsmitteln wird nicht beschrieben.

EP 643,042 B1 offenbart die Stabilisierung monomerer Isocyanate (Diisocyanate), hergestellt nach einem phosgenfreien Verfahren, durch Einsatz primärer Antioxidanzien, sekundärer Antioxidanzien oder Säuren, respektive Kombinationen primärer Antioxidanzien mit Säuren, ggf. in Anwesenheit sekundärer Antioxidanzien, in Bezug auf die Reaktivität und Farbe bei der Trimerisierung von monomerem HDI. Als saure Stabilisatoren werden Monocarbonsäuren, organische Polycarbonsäuren, anorganische Säuren, Diester der Phosphorsäure oder phosphorigen Säure, Carbonsäurechloride, und anorganische Säurechloride genannt. Die Stabilisierung bezieht sich dabei auf monomere Isocyanate vor ihrer Oligomerisierung. Die Oligomerisierung findet bei hoher Temperatur statt und es erfolgt abschließend eine Destillation bei ca. 150-160 °C. Es ist bekannt (z.B. aus der EP 1238992), dass z.B. Monocarbonsäuren mit Isocyanaten bei höherer Temperatur reagieren, also in den Polyisocyanaten nach Destillation bei 150 °C nicht mehr in aktiver Form vorliegen.
Lagerstabilität in Bezug auf Flockulation von Polyisocyanaten in Feuchtigkeit enthaltenden Lösungsmitteln wird nicht beschrieben.

US 5,256,333 beschreibt eine Dreierkombination von Diarylamin, sterisch gehindertem Phenol und einem Pentarythroldiphosphit, insbesondere zur Stabilisierung von Polyetherpolyolen gegen Verfärbung, insbesondere bei höheren Temperaturen bei der Herstellung von Polyurethanschäumen. Spalte 1 verweist auf eine erhöhte Hydrolysestabilität insbesondere bei den Polyethern gegenüber Verwendung einfacher Phosphite statt Diphosphite.

Nachteilig an den beschriebenen Antiflockulanzien für Polyisocyanate in Lösungsmitteln ist, dass diese fast durchgängig Wasserfänger sind und demzufolge mindestens stöchiometrisch eingesetzt werden müssen, da sie im Wettbewerb mit einem massiven Überschuss Isocyanat-Gruppen stehen. Dies ist nicht nur unökonomisch sondern bewirkt auch Nebeneffekte wie die Reduktion der NCO-Werte der Mischungen. Ferner bilden sich bei der Hydrolyse der Wasserfänger Spaltprodukte die oft Isocyanatgruppen verbrauchen und zu Folgeprodukten führen, die die Lackeigenschaften beeinträchtigen können.

Para-toluolsulfonylisocyanat und Amin-haltige Verbindungen führen in den zu verwendenden Mengen zur Verfärbung der aliphatischen Isocyanaten. Dies ist insbesondere unerwünscht, wenn die Polyisocyanat-Komponente für refinish-Anwendungen in Kleingebinden gelagert wird und für die Anwendung in Klarlacken eingesetzt werden soll.
Triethylorthoformat ist gegenüber para-Toluolsulfonylisocyanat der weniger reaktivere Wasserfänger.

Aufgabe der vorliegenden Erfindung war es, ein Verfahren zur Verfügung zu stellen, mit dem Polyisocyanate während der Lagerung in Lösungsmitteln bei Exposition gegenüber (Luft-)Feuchtigkeit eine höhere Flockulationsstabilität bei Lagerung aufweisen.

Eine spezielle Problematik stellt sich beispielsweise für refinish-Anwendungen (Automobil-Reparatur), bei denen von langen Lagerzeiten der Produkte in kleinen Gebinden auszugehen ist. Hersteller der Polyisocyanatkomponente in Lösungsmittel geben ihren Kunden Garantien des Beibehaltens der Produkteigenschaften von einem halben Jahr. Durch Lagerung beim Hersteller, Zwischenlagerung bei globalem Transport oder Überlagerung beim Kunden verlängern sich diese Fristen. Bedingt durch kleinvolumige Gebinde und/oder deren wiederholtes Öffnen kommt Umgebungsfeuchtigkeit an die Polyisocyanatkomponente. Ebenfalls enthalten die verwendeten Lösungsmittel Spuren an Feuchtigkeit. Lagereffekte werden insbesondere in südostasiatischen Ländern durch hohe Luftfeuchtigkeit und hohe Lagertemperaturen verschärft. Generell ist bei allen Anwendungen bei denen längere Lagerzeiten, insbesondere in kleineren Gebinden, auftreten von der gleichen Problematik auszugehen, beispielsweise in manchen industriellen Anwendungen.

Die Aufgabe wurde gelöst durch die Verwendung von Additiven ausgewählt aus der Gruppe bestehend aus
a1) organischen Säuren mit einem pKs-Wert von unter 4,2, ausgewählt aus der Gruppe bestehend aus a1a) aromatischen Sulfonsäuren und a1b) ein- oder zweifach mit Alkoxy-, Mercapto- oder Alkylmercapto-substituierte zwei Kohlenstoffatome aufweisende Alkancarbonsäuren, ein- oder zweifach mit Halogen-, Alkoxy-, Mercapto- oder Alkylmercapto-substituierte, mindestens drei Kohlenstoffatome aufweisende Alkancarbonsäuren, Alkendicarbonsäuren oder Alkandicarbonsäuren
a2 Phosphiten der Formel 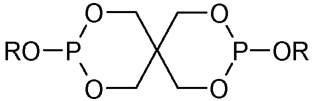 wobei R bevorzugt eine Aryl-Gruppe ist, die in 2, 4, und 6-Position wie folgt substituiert ist:
   Position 2: tert. Butyl, tert. Amyl
   Position 4: Wasserstoff, Alkyl, tert. Butyl oder tert. Amyl, und
   Position 6: Wasserstoff, Alkyl, tert. Butyl oder tert. Amyl,
   mit der Maßgabe, dass mindestens einer der Substituenten in Position 4 und 6 ungleich Wasserstoff ist,
a3) Phosphoniten der Formel (RO)₂P-X-P(RO)₂
   wobei R bevorzugt eine Aryl-Gruppe ist, die in 2, 4, und 6-Position wie folgt substituiert ist:
   Position 2: tert. Butyl, tert. Amyl
   Position 4: Wasserstoff, Alkyl, tert. Butyl oder tert. Amyl, und
   Position 6: Wasserstoff, Alkyl, tert. Butyl oder tert. Amyl,
   mit der Maßgabe, dass mindestens einer der Substituenten in Position 4 und 6 ungleich Wasserstoff ist, und
   X dabei eine Arylengruppe sei,
a4) sauren Phosphor-Derivaten, ausgewählt aus der Gruppe bestehend aus a4a) Mono- und Di-C₁- bis C₁₂-Alkylphosphaten, a4b) Mono- und Di-C₁- bis C₁₂-Alkylphosphonaten, a4c) Mono-Ci- bis C₁₂-Alkylphosphinaten und a4d) Alkylderivate von phosphorhaltigen Disäuren,
   zur Verringerung von Flockulation und/oder Niederschlagsbildung bei Exposition gegenüber (Luft-)Feuchtigkeit während der Lagerung von Polyisocyanatgemischen, welche mindestens ein Lösungsmittel enthalten.

Unter Polyisocyanaten im Sinne der Erfindung werden Polyisocyanate wie sie bei Ihrer Synthese anfallen, respektive Gemische dieser Polyisocyanate verstanden.
Diese umfassen die im Folgenden näher beschriebenen Verbindungen:
Die für die Herstellung der Polyisocyanate eingesetzten monomeren Isocyanate können aromatisch, aliphatisch oder cycloaliphatisch sein, bevorzugt aliphatisch oder cycloaliphatisch, was in dieser Schrift kurz als (cyclo)aliphatisch bezeichnet wird, besonders bevorzugt sind aliphatische Isocyanate.

Aromatische Isocyanate sind solche, die mindestens ein aromatisches Ringsystem enthalten, also sowohl rein aromatische wie auch araliphatische Verbindungen.

Cycloaliphatische Isocyanate sind solche, die mindestens ein cycloaliphatisches Ringsystem enthalten.

Aliphatische Isocyanate sind solche, die ausschließlich gerade oder verzweigte Ketten enthalten, also acyclische Verbindungen.

Bei den monomeren Isocyanaten handelt es sich bevorzugt um Diisocyanate, die genau zwei Isocyanatgruppen tragen. Es kann sich aber prinzipiell auch um Monoisocyanate mit einer Isocyanatgruppe handeln.

Es kommen prinzipiell auch höhere Isocyanate mit im Mittel mehr als 2 Isocyanatgruppen in Betracht. Dafür eignen sich beispielsweise Triisocyanate wie Triisocyanatononan, 2'-Isocyanatoethyl-(2,6-diisocyanatohexanoat), 2,4,6-Triisocyanatoluol, Triphenylmethantriisocyanat oder 2,4,4'-Triisocyanatodiphenylether oder die Gemische aus Di-, Tri- und höheren Polyisocyanaten, die beispielsweise durch Phosgenierung von entsprechenden Anilin/Formaldehyd-Kondensaten erhalten werden und Methylenbrücken aufweisende Polyphenylpolyisocyanate darstellen, insbesondere Triisocyanatononan und 2'-Isocyanatoethyl-(2,6-diisocyanatohexanoat.

Bei den monomeren Isocyanaten handelt es sich bevorzugt um Isocyanate mit 4 bis 20 C-Atomen. Beispiele für übliche Diisocyanate sind aliphatische Diisocyanate wie Tetramethylendiisocyanat, 1,5-Pentamethylendiisocyanat, Hexamethylendiisocyanat (1,6-Diisocyanatohexan), Octamethylendiisocyanat, Decamethylendiisocyanat, Dodecamethylendiisocyanat, Tetradecamethylendiisocyanat, Derivate des Lysindiisocyanats, (z.B. Methyl- oder Ethyl-2,6-diisocyanato-hexanoat), Trimethylhexandiisocyanat oder Tetramethylhexandiisocyanat, cycloaliphatische Diisocyanate wie 1,4-, 1,3- oder 1,2-Diisocyanatocyclohexan, 4,4'- oder 2,4'-Di(isocyanatocyclo-hexyl)methan, 1-Isocyanato-3,3,5-trimethyl-5-(isocyanatomethyl)cyclohexan (Isophorondiisocyanat), 1,3- oder 1,4-Bis(isocyanatomethyl)cyclohexan oder 2,4-, oder 2,6-Diisocyanato-1-me-thylcyclohexan sowie 3 (bzw. 4), 8 (bzw. 9)-Bis(isocyanatomethyl)-tricyclo[5.2.1.02.6]decan-Isomerengemische, sowie aromatische Diisocyanate wie 2,4- oder 2,6-Toluylendiisocyanat und deren Isomerengemische, m- oder p-Xylylendiisocyanat, 2,4'- oder 4,4'-Diisocyanatodiphenyl-methan und deren Isomerengemische, 1,3- oder 1,4-Phenylendiisocyanat, 1-Chlor-2,4-phenyl-endiisocyanat, 1,5-Naphthylendiisocyanat, Diphenylen-4,4'-diisocyanat, 4,4'-Diisocyanato-3,3'-dimethyldiphenyl, 3-Methyldi-phenylmethan-4,4'-diisocyanat, Tetramethylxylylendiisocyanat, 1,4-Diisocyanatobenzol oder Diphenylether-4,4'-diisocyanat.

Besonders bevorzugt sind 1,6-Hexamethylendiisocyanat, 1,3-Bis(isocyanatomethyl)cyclohexan, Isophorondiisocyanat und 4,4'- oder 2,4'-Di(isocyanatocyclohexyl)methan, ganz besonders bevorzugt sind Isophorondiisocyanat und 1,6-Hexamethylendiisocyanat, insbesondere bevorzugt ist 1,6-Hexamethylendiisocyanat.

Es können auch Gemische der genannten Isocyanate vorliegen.

Isophorondiisocyanat liegt zumeist als ein Gemisch, und zwar der cis- und trans-Isomere vor, in der Regel im Verhältnis von ca. 60:40 bis 90:10 (w/w), bevorzugt von 70:30-90:10. Dicyclohexylmethan-4,4'-diisocyanat kann ebenfalls als Gemisch der verschiedenen cis- und trans-Isomere vorliegen.

Für die vorliegende Erfindung können sowohl solche Diisocyanate eingesetzt werden, die durch Phosgenierung der korrespondierenden Amine erhalten werden, als auch solche, die ohne die Verwendung von Phosgen, d. h. nach phosgenfreien Verfahren, hergestellt werden. Nach Angaben der EP-A-0 126 299 (US 4 596 678), EP-A-126 300 (US 4 596 679) und EP-A-355 443 (US 5 087 739) beispielsweise können (cyclo)aliphatische Diisocyanate, z.B. wie 1,6-Hexamethylendiisocyanat (HDI), isomere aliphatische Diisocyanate mit 6 Kohlenstoffatomen im Alkylenrest, 4,4'- oder 2,4'-Di(isocyanatocyclohexyl)methan und 1-Isocyanato-3-isocyanato-methyl-3,5,5-trimethyl-cyclohexan (Isophorondiisocyanat bzw. IPDI) hergestellt werden durch Umsetzung der (cyclo)aliphatischen Diamine mit beispielsweise Harnstoff und Alkoholen zu (cyclo)-aliphatischen Biscarbaminsäureestern und deren thermische Spaltung in die entsprechenden Diisocyanate und Alkohole. Die Synthese erfolgt meist kontinuierlich in einem Kreislaufverfahren und optional in Gegenwart von N-unsubstituierten Carbaminsäureestern, Dialkylcarbonaten und anderen aus dem Reaktionsprozess zurückgeführten Nebenprodukten. So erhaltene Diisocyanate weisen in der Regel einen sehr geringen oder sogar nicht messbaren Anteil an chlorierten Verbindungen auf, was beispielsweise in Anwendungen in der Elektronikindustrie vorteilhaft ist.

In einer Ausführungsform der vorliegenden Erfindung weisen die eingesetzten Isocyanate an hydrolysierbarem Chlor weniger als 100 ppm auf, bevorzugt weniger als 50 ppm, insbesondere weniger als 30 ppm und speziell weniger als 20 ppm. Dies kann beispielsweise gemessen werden durch die ASTM-Vorschrift D4663-98. Die Gehalte an gesamtem Chlor liegen beispielsweise bei unter 1000 ppm, bevorzugt unter 800 ppm und besonders bevorzugt unter 500 ppm (ermittelt per argentometrischer Titration nach Hydrolyse).

Selbstverständlich können auch Gemische aus solchen monomeren Isocyanaten, die durch Umsetzung der (cyclo)aliphatischen Diamine mit beispielsweise Harnstoff und Alkoholen und Spaltung der erhaltenen (cyclo)aliphatischen Biscarbaminsäureester erhalten worden sind, mit solchen Diisocyanaten, die durch Phosgenierung der korrespondierenden Amine erhalten worden sind, eingesetzt werden.

Die Polyisocyanate, zu denen die monomeren Isocyanate oligomerisiert werden können, sind in der Regel wie folgt charakterisiert:
Die mittlere NCO Funktionalität solcher Verbindungen beträgt in der Regel mindestens 1,8 und kann bis zu 8 betragen, bevorzugt 2 bis 5 und besonders bevorzugt 2,4 bis 4.

Der Gehalt an Isocyanatgruppen nach der Oligomerisierung, berechnet als NCO = 42 g/mol, beträgt, wenn nicht anders angegeben, in der Regel von 5 bis 25 Gew%.

Bevorzugt handelt es sich bei den Polyisocyanaten um folgende Verbindungen:
1) Isocyanuratgruppen aufweisende Polyisocyanate von aromatischen, aliphatischen und/oder cycloaliphatischen Diisocyanaten. Besonders bevorzugt sind hierbei die entsprechenden aliphatischen und/oder cycloaliphatischen Isocyanato-Isocyanurate und insbesondere die auf Basis von Hexamethylendiisocyanat und Isophorondiisocyanat. Bei den dabei vorliegenden Isocyanuraten handelt es sich insbesondere um Tris-isocyanatoalkyl- bzw. Tris-isocyanatocycloalkyl-Isocyanurate, welche cyclische Trimere der Diisocyanate darstellen, oder um Gemische mit ihren höheren, mehr als einen Isocyanuratring aufweisenden Homologen. Die Isocyanato-Isocyanurate haben im Allgemeinen einen NCO-Gehalt von 10 bis 30 Gew.-%, insbesondere 15 bis 25 Gew.-% und eine mittlere NCO-Funktionalität von 2,6 bis 8.
   Die Isocyanuratgruppen aufweisenden Polyisocyanate können in geringerem Umfang auch Urethan- und/oder Allophanat-Gruppen enthalten, bevorzugt mit einem Gehalt gebundenen Alkohols von kleiner 2 % bezogen auf das Polyisocyanat.
2) Uretdiongruppen aufweisende Polyisocyanate mit aromatisch, aliphatisch und/oder cycloaliphatisch gebundenen Isocyanatgruppen, vorzugsweise aliphatisch und/oder cycloaliphatisch gebundenen und insbesondere die von Hexamethylendiisocyanat oder Isophorondiisocyanat abgeleiteten. Bei Uretdiondiisocyanaten handelt es sich um cyclische Dimerisierungsprodukte von Diisocyanaten.
   Die Uretdiongruppen aufweisenden Polyisocyanate werden häufig im Gemisch mit anderen Polyisocyanaten, insbesondere den unter 1) genannten, erhalten. Uretdiongruppen aufweisende Polyisocyanate weisen üblicherweise Funktionalitäten von 2 bis 3 auf. Dies umfasst auch Uretdion-/lsocyanurat-Gemische beliebiger Zusammensetzung, insbesondere mit einem Gehalt an monomerem Uretdion (Dimer) von 1-40%, insbesondere 3-15, insbesondere 5-10 %.
   Dazu können die Diisocyanate unter Reaktionsbedingungen umgesetzt werden, unter denen sowohl Uretdiongruppen als auch die anderen Polyisocyanate gebildet werden, oder zunächst die Uretdiongruppen gebildet und diese anschließend zu den anderen Polyisocyanaten umgesetzt werden oder die Diisocyanate zunächst zu den anderen Polyisocyanaten und diese anschließend zu Uretdiongruppen-haltigen Produkten umgesetzt werden.
3) Biuretgruppen aufweisende Polyisocyanate mit aromatisch, cycloaliphatisch oder aliphatisch gebundenen, bevorzugt cycloaliphatisch oder aliphatisch gebundenen Isocyanat-gruppen, insbesondere Tris-(6-isocyanatohexyl)-biuret oder dessen Gemische mit seinen höheren Homologen. Diese Biuretgruppen aufweisenden Polyisocyanate weisen im Allgemeinen einen NCO-Gehalt von 18 bis 24 Gew.-% und eine mittlere NCO-Funktionalität von 2,8 bis 6 auf.
4) Urethan- und/oder Allophanatgruppen aufweisende Polyisocyanate mit aromatisch, aliphatisch oder cycloaliphatisch gebundenen, bevorzugt aliphatisch oder cycloaliphatisch gebundenen Isocyanatgruppen, wie sie beispielsweise durch Umsetzung von überschüssigen Mengen an Diisocyanat, beispielsweise Hexamethylendiisocyanat oder Isophorondiisocyanat, mit ein- oder mehrwertigen Alkoholen. Diese Urethan- und/oder Allophanat-gruppen aufweisenden Polyisocyanate haben im Allgemeinen einen NCO-Gehalt von 12 bis 24 Gew.-% und eine mittlere NCO-Funktionalität von 2,0 bis 4,5. Solche Urethan- und/oder Allophanatgruppen aufweisenden Polyisocyanate können unkatalysiert oder bevorzugt in Gegenwart von Katalysatoren, wie beispielsweise Ammoniumcarboxylaten oder -hydroxiden, oder Allophanatisierungskatalysatoren, z.B. Wismut-, Kobalt-, Cäsium-, Zn-(II)- oder Zr-(IV)-Verbindungen, jeweils in Anwesenheit von ein-, zwei- oder mehrwertigen, bevorzugt einwertigen Alkoholen, hergestellt werden.
   Diese Urethan- und/oder Allophanatgruppen aufweisenden Polyisocyanate treten häufig in Mischformen mit den unter 1) genannten Polyisocyanaten auf.
5) Oxadiazintriongruppen enthaltende Polyisocyanate, vorzugsweise von Hexamethylendiisocyanat oder Isophorondiisocyanat abgeleitet. Solche Oxadiazintriongruppen enthaltenden Polyisocyanate sind aus Diisocyanat und Kohlendioxid zugänglich.
6) Iminooxadiazindiongruppen enthaltende Polyisocyanate, vorzugsweise von Hexamethylendiisocyanat oder Isophorondiisocyanat abgeleitet. Solche Iminooxadiazindiongruppen enthaltenden Polyisocyanate sind aus Diisocyanaten mittels spezieller Katalysatoren herstellbar.
7) Hyperverzweigte Polyisocyanate, wie sie beispielsweise bekannt sind aus der DE-A1 10013186 oder DE-A1 10013187.
8) Polyurethan-Polyisocyanat-Präpolymere, aus Di- und/oder Polyisocyanaten mit Alkoholen.
9) Polyharnstoff-Polyisocyanat-Präpolymere.
10) Die Polyisocyanate 1)-9, bevorzugt 1), 3), 4) und 6) können nach deren Herstellung in Biuretgruppen- oder Urethan-/Allophanat-Gruppen aufweisende Polyisocyanate mit aromatisch, cycloaliphatisch oder aliphatisch gebundenen, bevorzugt (cyclo)aliphatisch gebundenen Isocyanatgruppen, überführt werden. Die Bildung von Biuretgruppen erfolgt beispielsweise durch Zugabe von Wasser oder Umsetzung mit Aminen. Die Bildung von Urethan- und/oder Allophanatgruppen erfolgt durch Umsetzung mit ein-, zwei- oder mehrwertigen, bevorzugt einwertigen Alkoholen, optional in Gegenwart von geeigneten Katalysatoren. Diese Biuret- oder Urethan-/Allophanatgruppen aufweisenden Polyisocyanate weisen im Allgemeinen einen NCO-Gehalt von 10 bis 25 Gew.-% und eine mittlere NCO-Funktionalität von 3bis 8 auf.
11) Hydrophil modifizierte Polyisocyanate, d.h. Polyisocyanate, die neben den unter 1-10 beschriebenen Gruppen solche enthalten, die formal durch Addition von Molekülen mit NCO-reaktiven Gruppen und hydrophilierenden Gruppen an die Isocyanatgruppen obiger Moleküle entstehen. Bei letzteren handelt es sich um nichtionische Gruppen wie Alkyl-Polyethylenoxid und/oder ionische, welche von Phosphorsäure, Phosphonsäure, Schwefelsäure oder Sulfonsäure, bzw. ihren Salzen abgeleitet sind.
12) Modifizierte Polyisocyanate für Dual Cure Anwendungen, d.h. Polyisocyanate, die neben den unter 1-11 beschriebenen Gruppen solche enthalten, die formal durch Addition von Molekülen mit NCO-reaktiven Gruppen und durch UV- oder aktinische Strahlung vernetzbare Gruppen an die Isocyanatgruppen obiger Moleküle entstehen. Bei diesen Molekülen handelt es sich beispielsweise um Hydroxyalkyl(meth)acrylate und andere Hydroxy-Vinylverbindungen.

Die oben aufgeführten Diisocyanate oder Polyisocyanate können auch zumindest teilweise in blockierter Form vorliegen.

Zur Blockierung eingesetzte Verbindungsklassen sind beschrieben in D. A. Wicks, Z. W. Wicks, Progress in Organic Coatings, 36, 148-172 (1999), 41, 1-83 (2001) sowie 43, 131-140 (2001).

Beispiele für zur Blockierung eingesetzte Verbindungsklassen sind Phenole, Imidazole, Triazole, Pyrazole, Oxime, N-Hydroxyimide, Hydroxybenzoesäureester, sekundäre Amine, Lactame, CH-acide cyclische Ketone, Malonsäureester oder Alkylacetoacetate.

In einer bevorzugten Ausführungsform der vorliegenden Erfindung ist das Polyisocyanat ausgewählt aus der Gruppe bestehend aus Isocyanuraten, Biureten, Urethanen und Allophanaten, bevorzugt aus der Gruppe bestehend aus Isocyanuraten, Urethanen und Allophanaten, besonders bevorzugt handelt es sich um ein isocyanuratgruppenhaltiges Polyisocyanat.

In einer besonders bevorzugten Ausführungsform handelt es sich bei dem Polyisocyanat um Isocyanuratgruppen enthaltende Polyisocyanate von 1,6-Hexamethylendiisocyanat.

In einer weiteren bevorzugten Ausführungsform handelt es sich bei dem Polyisocyanat um ein Gemisch von Isocyanuratgruppen enthaltenden Polyisocyanaten, ganz besonders bevorzugt von 1,6-Hexamethylendiisocyanat und von Isophorondiisocyanat.

In einer besonders bevorzugten Ausführungsform handelt es sich bei dem Polyisocyanat um ein Gemisch enthaltend niedrigviskose Polyisocyanate, bevorzugt Isocyanuratgruppen enthaltende Polyisocyanate, mit einer Viskosität von 600-1500 mPa*s, insbesondere unter 1200 mPa*s, niederviskose Urethane und/oder Allophanate mit einer Viskosität von 200-1600 mPa*s, insbesondere 600-1500 mPa*s, und/oder Iminooxadiazindiongruppen enthaltende Polyisocyanate.

In dieser Schrift wird die Viskosität bei 23 °C gemäß DIN EN ISO 3219/A.3 in einem Kegel-Platte-System mit einem Geschwindigkeitsgefälle von 1000 s⁻¹ angegeben, falls nicht anders vermerkt.

Das Verfahren zur Herstellung der Polyisocyanate kann erfolgen, wie in WO 2008 / 68198 beschrieben, dort besonders von Seite 20, Zeile 21 bis Seite 27, Zeile15, was hiermit durch Bezugnahme Bestandteil der vorliegenden Anmeldung sei.

Die Reaktion kann beispielsweise abgebrochen werden, wie dort von Seite 31, Zeile 19 bis Seite 31, Zeile 31 beschrieben und die Aufarbeitung erfolgen wie dort beschrieben von Seite 31, Zeile 33 bis Seite 32, Zeile 40, was hiermit jeweils durch Bezugnahme Bestandteil der vorliegenden Anmeldung sei.

Die Reaktion kann alternativ erfolgen wie in der WO 2005 / 087828 für Ammonium-alphahydroxycarboxylat-Katalysatoren beschrieben. Die in WO 2005/87828, Seite 3, Zeile 29 bis Seite 6, Zeile 7 beschriebenen Ammonium α-Hydroxycarboxylate seien hiermit ausdrücklich Bestandteil der vorliegenden Offenbarung. Die Reaktion kann beispielsweise abgebrochen werden, wie dort von Seite 11, Zeile 12 bis Seite 12, Zeile 5 beschrieben, was hiermit durch Bezugnahme Bestandteil der vorliegenden Anmeldung sei.

Die Reaktion kann alternativ erfolgen wie in der CN 10178994A oder CN 101805304 beschrieben.

Bei thermisch labilen Katalysatoren ist es weiterhin auch möglich, die Reaktion abzubrechen durch Erhitzen des Reaktionsgemischs auf eine Temperatur oberhalb von mindestens 80°C, bevorzugt mindestens 100 °C, besonders bevorzugt mindestens 120 °C. Dafür reicht in der Regel bereits die Erwärmung des Reaktionsgemischs, wie sie zur Abtrennung des unumgesetzten Isocyanats durch Destillation in der Aufarbeitung erforderlich ist.

Sowohl bei thermisch nicht-labilen als auch bei thermisch labilen Katalysatoren besteht die Möglichkeit, durch Zugabe von Deaktivatoren die Reaktion bei niedrigeren Temperaturen abzubrechen. Deaktivatoren können auch stöchiometrisch im Unterschuss zum Katalysator zugegeben werden, wenn der Katalysator zumindest partiell thermisch zerstört wird oder das Produkt bei nachfolgender Lagerung in der Viskosität stabil ist (z.B. bei Lagerung in der 100%-Form über 10 Wochen bei 80 °C unter Stickstoff seine Viskosität nicht mehr als verdreifacht). Geeignete Deaktivatoren sind beispielsweise Chlorwasserstoff, Phosphorsäure, organische Phosphate, wie Dibutylphosphat oder Diethylhexylphosphat, und Carbamate wie Hydroxyalkylcarbamat.

Diese Verbindungen werden pur oder verdünnt in geeigneter Konzentration, die zum Reaktionsabbruch erforderlich ist, zugegeben.

Erfindungsgemäß antiflockulierend wirkende Additive:
a1) Organische Säuren mit pKs-Werten kleiner als 4,2
a1a) Bevorzugte Beispiele sind im Sinne der Erfindung sind aromatische Sulfonsäuren mit pKs-Werten kleiner als 4,2, besonders bevorzugt Benzol- oder Naphthalinderivate, insbesondere alkylierte Benzol oder Naphthalinderivate.

Beispiele bevorzugter Sulfonsäuren umfassen 4-Alkylbenzolsulfonsäuren mit Alkylresten aus 6 bis 12 C-Atomen, bevorzugt in ortho- oder para-Position, bevorzugt para-Position zur Sulfonsäuregruppe, wie beispielsweise 4-Hexylbenzolsulfonsäure, 4-Octylbenzolsulfonsäure, 4-Decylbenzolsulfonsäure oder 4-Dodecylbenzolsulfonsäure. Hierbei kann es sich in prinzipiell bekannter Art und Weise auch um technische Produkte handeln, welche eine Verteilung verschiedener Alkylreste unterschiedlicher Länge aufweisen.

Als besonders bevorzugte Säuren genannt seien:
- Benzolsulfonsäure
- Para-toluolsulfonsäure
- Para-ethylbenzolsulfonsäure
- Dodecylbenzolsulfonsäure
- Bisnonylnaphthalinsulfonsäure
- Bisnonylnaphthalinbissulfonsäure
- Bisdodecylnaphthalinsulfonsäure
- Nacure® XC-C210 (hydrophober Säurekatalysator nicht angegebener Struktur der Firma King Industries)

### a1b)

Weitere bevorzugte Säuren im Sinne der Erfindung sind ein- oder zweifach mit Alkoxy-, Mercapto- oder Alkylmercapto-substituierte, zwei Kohlenstoffatome aufweisende Alkancarbonsäuren, ein- oder zweifach mit Halogen-, Alkoxy-, Mercapto- oder Alkylmercapto-substituierte, mindestens drei Kohlenstoffatome aufweisende Alkancarbonsäuren, Alkendicarbonsäuren oder Alkandicarbonsäuren, bevorzugt die genannten substituierten Alkancarbonsäuren mit einem pK_{S}-Wert unter 4,2.

Zwei oder mindestens drei Kohlenstoffatome aufweisende Alkancarbonsäuren sind beispielsweise solche mit 2 bis 8, bevorzugt 2 bis 6, besonders bevorzugt 2 bis 4 und ganz besonders bevorzugt 2 oder 3 Kohlenstoffatomen. Diese Alkancarbonsäuren können dabei geradkettig oder verzweigt sein.

Besonders zu erwähnen sind als derartige Alkancarbonsäuren als Grundkörper Essigsäure, Propionsäure, Buttersäure, iso-Buttersäure und Capronsäure.

Die Grundkörper dieser Alkancarbonsäuren sind ein- oder zweifach, bevorzugt einfach mit Halogen-, Alkoxy-, Mercapto- oder Alkylmercapto-substituiert, wobei die Substituenten gleich oder verschieden sein können.

Halogen ist dabei Chlor oder Brom, bevorzugt Chlor. Halogen-essigsäurederivate sind nicht erfindungsgemäß.

Mit Alkoxy ist dabei C₁- bis C₁₂-Alkyloxy gemeint, bevorzugt C₁- bis C₈-Alkyloxy, besonders bevorzugt C₁- bis C₄-Alkyloxy und ganz besonders bevorzugt C₁- bis C₂-Alkyloxy.

Beispiele sind Methoxy, Ethoxy, iso-Propyloxy, n-Propyloxy, n-Butyloxy, iso-Butyloxy, sek-Butyloxy, tert-Butyloxy, n-Hexyloxy, n-Heptyloxy, n-Octyloxy, n-Decyloxy, n-Dodecyloxy, 2-Ethylhexyloxy und 2-Propylheptyloxy.
Unter Mercapto wird dabei eine Thiolgruppe (-SH) verstanden.

Mit Alkylmercapto ist dabei C₁- bis C₁₂- Alkylmercapto gemeint, bevorzugt C₁- bis C₈- Alkylmercapto, besonders bevorzugt C₁- bis C₄- Alkylmercapto und ganz besonders bevorzugt C₁- bis C₂- Alkylmercapto. Beispiele dafür sind die schwefel-analogen Verbindungen der oben angeführten Alkoxygruppen. Auch Arylthio-, insbesondere Phenylthiogruppen sind möglich.

Unter Aryl wird in dieser Schrift 6 bis 12 Kohlenstoffatome aufweisende, einbindige aromatische Reste verstanden, die optional, wenn auch weniger bevorzugt, substituiert sein können. Beispiele dafür sind Phenyl, α-Naphthyl und β-Naphthyl.

Unter den Substituenten sind Halogen, Alkoxy und Mercapto bevorzugt, besonders bevorzugt sind Halogen und Alkoxy und besonders bevorzugt Halogen.

Besonders bevorzugt sind solche substituierten Alkancarbonsäuren, die mindestens einen Substituenten in α- oder β-Position zur Carboxylgruppe tragen, wobei die β-Position selbstverständlich nur bei Alkancarbonsäuren mit mindestens 3 Kohlenstoffatomen in Betracht kommt.

Beispiele für besonders bevorzugte Säuren im Sinne der Erfindung sind 2- oder 3-Chloralkancarbonsäuren mit mindestens drei, bevorzugt drei bis sechs, besonders bevorzugt drei bis vier und ganz besonders bevorzugt drei Kohlenstoffatomen.

Beispiele für eine Alkendicarbonsäure sind Maleinsäure und Fumarsäure.

Ein Beispiel für eine Alkandicarbonsäure ist das formale Addukte von Salzsäure an Maleinsäure, also chlorsubstituierte Bernsteinsäure.

Besonders bevorzugte Beispiele für erfindungsgemäße Säuren a1b) sind
- 3-Chlorpropionsäure und
- 2-Chlorpropionsäure.

Diese sind sehr gute Antiflockulanzien, auch im Vergleich zu Säuren von vergleichbarem pKs-Wert wie Methoxyessigsäure. Es ist nicht auszuschließen, ohne sich auf einen Wirkmechanismus beschränken zu wollen, dass Chlorpropionsäuren in basischer Umgebung unter Abspaltung von Salzsäure reagieren, also latent verkappte Salzsäure mit niedrigerem pKs-Wert darstellen.

**pKs-Werte: Die folgende Tabelle zeigt die pKs-Werte einiger Säuren:**

| Säure | pKs-Wert |
|---|---|
| **Nicht erfindungsgemäß** | |
| Ethylhexansäure | 4,8 |
| Essigsäure | 4,76 |
| Pivalinsäure | |
| Acrylsäure | 4,3 |
| Phenylessigsäure | 4,31 |
| Benzoesäure | 4,2 |
| Ameisensäure | 3,8 |
| Chloressigsäure | 2,86 |
| Dichloressigsäure | 1,29 |
| Trichloressigsäure | 0,65 |
| Trifluoressigsäure | 0,2 |
| Methansulfonsäure | -1,9 |
| Trifluormethansulfonsäure | -5,5 |
| | |

| **Erfindungsgemäß** | |
|---|---|
| 3-Chlorpropionsäure | 4,1 |
| Mercaptoessigsäure | 3,55 |
| Methoxyessigsäure | 3,48 |
| Diethylhexylphosphat | 3,2 |
| Fumarsäure | 3,0 |
| 2-Chlorpropionsäure | 2,8 |
| Nacure® 5076 der Firma King Industries (aromatische Sulfonsäure) | -3 |

| | |
|---|---|
| Überraschenderweise wirken auch einige Säuren mit pKs-Wert unter 4,2 nicht signifikant antiflockulierend, z.B. Trichloressigsäure, Trifluoressigsäure, Methansulfonsäure, Trifluormethansulfonsäure. | |

Nicht erfindungsgemäß sind unsubstitutierte oder kürzere Alkylcarbonsäuren wie Ameisensäure Essigsäure, Ethylhexansäure, Pivalinsäure, Acrylsäure sowie Arylcarbonsäuren wie Benzoesäure.

Säuren werden zum Teil bei der Trimerisierung und Allophanatisierung (Isocyanuratisierung) als Stopper oder bei der Biuretisierung als Katalysatoren eingesetzt, und liegen damit ggf. inhärent in den Produkten vor. Explizit im Stand der Technik genannt werden als Isocyanuratisierungsstopper z.B. Salzsäure, Säurechloride, Phosphorsäure und phosphorige Säure und ihre Derivate wie Dibutylphosphat, Di-(2-ethylhexyl)-phosphat und Dibutylphosphit, sowie Sulfonsäuren wie para-Toluolsulfonsäure und ihre Methyl- und Ethylester (EP 330966).
Explizit als Biuretisierungskatalysatoren werden Dialkylphosphate genannt (EP 918809).
Dabei wird jedoch kein Hinweis darauf gegeben, dass solcherart gestoppte Produkte besondere antiflockulative Vorteile beim Einsatz der Polyisocyanate in Lösungen haben.

Als gezielt zugegebene Antiflockulanzien können die erfindungsgemäß bevorzugten Säuren universal und unabhängig vom Herstellungsverfahren eingesetzt werden, z.B. auch bei Silicumbasierten Isocyanuratisierungskatalysator, wie Silazanen, mit Stoppung durch Alkohole wie n-Butanol, z.B. wie in den Schriften EP 57653 B1 und EP 89297 B1 beschrieben, oder bei thermischer Stoppung bei der Isocyanuratisierung und Allophanatisierung mit Ammoniumcarboxylaten oder bei Uretdionen unter Phosphinkatalyse und chemischer Stoppung mit Schwefel Insbesondere bei der Isocyanuratisierung mit thermischer Stoppung, also thermischer Desaktivierung des verwendeten Katalysators, zeigt das erfindungsgemäße Stabilisierungsverfahren einen besonderen Vorteil. Derartige Katalysatoren sind insbesondere quartäre 2-Hydroxyalkylammoniumsalze.

2-Chlorpropionsäure ist als chemischer Stopper bei der Isocyanuratisierung mit einem Trimethylbenzylammoniumhydroxid- oder einem Tetralkylammonium-Hydroxycarboxylat-Katalysator bei 65 °C in stöchiometrischer Menge nicht ausreichend stark, so dass sich das Produkt zwar herstellen lässt, aber dann in einen gelartigen Zustand übergeht, vermutlich infolge unverminderter Polymerisation von Isocyanatgruppen durch weitergehende Katalyse des nicht vollständig desaktivierten Katalysators.
2-Chlorpropionsäure ist bei Zugabe zu einem stabilen Isocyanurat dagegen ein sehr gutes Antiflockulanz.

### a2) Mehrkernige Phosphite

Phosphite sind bekannt als sekundäre Antioxidanzien, die insbesondere in Synergie mit primären Antioxidanzien wie sterisch gehinderten Phenolderivaten gegen Oxidation der von ihnen stabilisierten Produkte sorgen, und damit zum Beispiel einen Farbdrift reduzieren. Überraschenderweise wurde gefunden, dass bestimmte Phosphite auch antiflockulierende Wirkung bei Polyisocyanaten in Lösungsmitteln haben. Die antiflockulierende Wirkung tritt alleine und optional in Kombination mit sterisch gehinderten Phenolen auf, aber nicht bei Monophosphiten (vgl. Versuchsreihe 2, Vgl.-Bsp. 5 u.a.). Als gemeinsames Strukturmerkmal der antiflockulierend wirkenden Phosphite wurde gefunden, dass diese Oligophosphite, insbesondere Diphosphite, insbesondere Pentaerythroldiphosphite sind (s. die folgende Abbildung). Im Sinne der Erfindung sind also Pentaerythroldiphosphite und vergleichbare Strukturen respektive entsprechender Wirkung gemeint. In einer erfindungsgemäßen Form sind dies Bisaryl-pentaerythroldiphospite. Insbesondere sind dies solche, in denen die Arylgruppen substituierte Phenolgruppen mit mindestens zwei verzweigten, bevorzugt tertiären aliphatischen Substituenten sind. Triphenylphosphit als das am weitesten in Polyisocyanaten oder deren Mischungen und Lösungen eingesetzte Phosphit hat dabei keine Wirkung (vgl. Versuchsreihe 2, Vgl.-Bsp. 5 u.a.). Im Gegenteil wird die Flockulationswirkung noch erhöht.
Zum Teil sind diese sterisch angespannten Diphosphite als hydrolyseempfindlich bekannt, wie das Irgafos® 126. Überraschenderweise wirken sie jedoch schon in deutlich unterstöchiometrischen Mengen sehr stark antiflockulierend. 600 ppm Irgafos® 126 bezogen auf 30% Polyisocyanat in Lösungsmittel entsprechen bei äquimolarer Hydrolyse 8 ppm Wasser auf Lösungsmittel, was gegenüber den 800 ppm Wasser im Lösungsmittel nachfolgender Beispiele vernachlässigbar ist. Der antiflockulierende Effekt kann also nicht ausschlaggebend auf die Eigenschaft als Wasserfänger zurückgeführt werden.

Diphosphite sind beispielsweise Verbindungen der folgenden Struktur auf Basis Pentaerythrol: 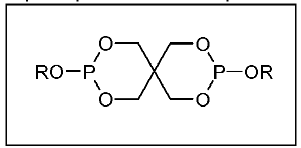 Wobei R eine Aryl-Gruppe ist, die in 2, 4, und 6-Position wie folgt substituiert sein kann:
Position 2: tert. Butyl, tert. Amyl
Position 4: Wasserstoff, Alkyl, tert. Butyl oder tert. Amyl, und
Position 6: Wasserstoff, Alkyl, tert. Butyl oder tert. Amyl,
mit der Maßgabe, dass mindestens einer der Substituenten in Position 4 und 6 der Arylgruppe ungleich Wasserstoff ist.

Bevorzugte Beispiele sind:
- "9228 = Doverphos® S-9228 = 3,9-Bis(2,4-di-tert-butylphenoxy)-2,4,8,10-tetraoxa-3,9-diphosphaspiro[5.5]undecan (CAS = 154862-43-8)
- 3,9-Bis(2,6-di-tert-butyl-4-methylphenoxy)-2,4,8,10-tetraoxa-3,9-diphosphaspiro(5.5)undecan (CAS 80693-00-1) = ADK Stab PEP-3
- Irgafos® 126 = Bis-(2,4-di-tert-butylphenyl)-pentaerythrol-diphosphit (CAS 26741-53-7)
- Weston® 618 = Doverphos® S 680 = Dioctadecyl-pentaerythritol-diphosphit (CAS 3806-34-6)
- ADK Stab PEP 36 (CAS 80693-00-1)
- Bis(2,4-di-tert.-butyl-phenyl)-pentaerythroldiphosphit; 3,9-Bis-(2,4-bis-(1,1-dimethylethyl)phenoxy)-2,4,8,10-tetraoxa-3,9-diphosphaspiro-(5,5)-undekan = ADK Stab PEP 24 = Doverphos® S 9432 = Weston® 626

### a3) Phosphonite

Phosphonite sind bekannt als sekundäre Antioxidanzien, die insbesondere in Synergie mit primären Antioxidanzien wie sterisch gehinderten Phenolderivaten gegen Oxidation der von ihnen stabilisierten Produkte sorgen, und damit zum Beispiel einen Farbdrift reduzieren. Überraschenderweise wurde gefunden, dass Phosphonite auch antiflockulierende Wirkung in aliphatischen Lösungsmitteln haben. Phosphonite als Stabilisatoren gegen Vergilbung sind beispielsweise in der WO 2008/116894 beschrieben. Diese, und in der US 4075163 beschriebenen Verbindungen seien hiermit als Bestandteil dieser Erfindung aufgenommen. Die erfindungsgemäß einsetzbaren Phosphonite haben folgende Struktur:

- (RO)₂P-X-P(OR)₂

Wobei R eine Aryl-Gruppe ist, die in 2, 4, und 6-Position wie folgt substituiert sein kann:
Position 2: tert. Butyl, tert. Amyl
Position 4: Wasserstoff, Alkyl, tert. Butyl oder tert. Amyl, und
Position 6: Wasserstoff, Alkyl, tert. Butyl oder tert. Amyl,
mit der Maßgabe, dass mindestens einer der Substituenten in Position 4 und 6 der Arylgruppe ungleich Wasserstoff ist.

X sei dabei eine Arylengruppe, also ein zweiwertiger, 6 bis 12 Kohlenstoffatome aufweisender organischer Rest, beispielsweise Phenylen, Naphthylen oder Biphenylen

### Besonders bevorzugt ist.

- Irgafos® P-EPQ = formales Reaktionsprodukt von Phosphortrichlorid mit 1,1'- Biphenyl- und 2,4-bis-(1,1-dimethylethyl)-phenol (siehe folgende Abbildung mit R = H. R = Methyl ist ebenfalls bevorzugt.) 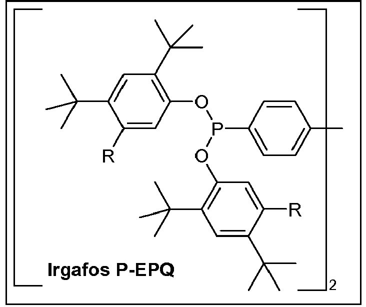 Irgafos® P-EPQ kann in Anwesenheit von Feuchtigkeit und bevorzugt unter aciden Bedingungen hydrolysieren, und damit formal als Wasserfänger agieren. Diese Reaktion erfolgt autokatalytisch, da mit der Hydrolyse weitere Säure generiert wird. Überraschender Weise wirkt Irgafos® P-EPQ schon in deutlich unterstöchiometrischer Menge antiflockulierend. 600 ppm Irgafos® P-EPQ bezogen auf 30% Polyisocyanat in Lösungsmittel entsprechen bei äquimolarer Hydrolyse 5 ppm Wasser, was gegenüber den 800 ppm Wasser im Lösungsmittel vieler Versuche vernachlässigbar ist. Der antiflockulierende Effekt kann also nicht ausschlaggebend auf die Eigenschaft als Wasserfänger zurückgeführt werden und ist damit überraschend.

### a4) Weitere Beispiele sind saure Phosphor-Derivate unabhängig von der Oxidationsstufe und pKs:

a4a) Verbindungen a4a) sind Mono- und Di-C₁- bis C₁₂-Alkylphosphate und deren Gemischen, bevorzugt die Dialkylphosphate, besonders bevorzugt solche mit C₁- bis C₈-Alkylgruppen, ganz besonders bevorzugt mit C₂- bis C₈-Alkylgruppen und insbesondere solche mit C₄- bis C₈-Alkylgruppen.

Die Alkylgruppen in Dialkylphosphaten können dabei gleich oder verschieden sein, bevorzugt sind sie gleich.

Beispiele für C₁- bis C₁₂-Alkylgruppen sind Methyl, Ethyl, iso-Propyl, n-Propyl, n-Butyl, iso-Butyl, sek-Butyl, tert.-Butyl, n-Hexyl, n-Heptyl, n-Octyl, n-Decyl, n-Dodecyl, 2-Ethylhexyl und 2-Propylheptyl.

Dies sind insbesondere Mono- und Dialkylphosphate und deren Gemische wie
- Di-(ethylhexyl)-phosphat
- Di-n-butylphosphat
- Diethylphosphat
- Nacure® 4000 (früher Nacure® C 207), einem nicht näher definierten Alkylphosphorsäureester, der Firma King Industries
- Nacure® 4054, einem nicht näher definierten Alkylphosphorsäureester, der Firma King Industries
- Cycat® 296-9, einem nicht näher definierten Alkylphosphorsäureester, der Firma Cytec

Bevorzugt für den Einsatz in Polyisocyanaten ist der Einsatz dieser Verbindungen a4a) als 100%-iges Produkt oder in einem Lösungsmittel, welches nicht mit Isocyanat-Gruppen reagiert.
a4b) Beispiele niedervalenter phosphorhaltiger Verbindungen mit acidem Charakter sind Verbindungen a4b) Dialkylphosphonate und Dialkyldiphosphonate. 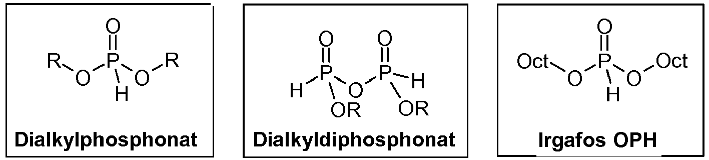
Beispiele dafür sind Mono- und Di-C₁- bis C₁₂-Alkylphosphonate und deren Gemischen, bevorzugt die Dialkylphosphonate, besonders bevorzugt solche mit C₁- bis C₈-Alkylgruppen, ganz besonders bevorzugt mit C₁- bis C₈-Alkylgruppen und insbesondere solche mit C₁-, C₂-, C₄- oder C₈-Alkylgruppen.

Die Alkylgruppen in Dialkylphosphonaten können dabei gleich oder verschieden sein, bevorzugt sind sie gleich.

Beispiele für C₁- bis C₁₂-Alkylgruppen sind Methyl, Ethyl, iso-Propyl, n-Propyl, n-Butyl, iso-Butyl, sek-Butyl, tert.-Butyl, n-Hexyl, n-Heptyl, n-Octyl, n-Decyl, n-Dodecyl, 2-Ethylhexyl und 2-Propylheptyl.

Die in der WO 2008/116895 beschriebenen Beispiele seien hiermit inhärenter Bestandteil dieses Patents. Als spezifische Beispiele seien explizit genannt:
- Phosphonsäuredioctylester, Phosphonsäuredi-n-octylester Irgafos® OPH (s. obige Abbildung)
- Phosphonsäuredi-(2-ethylhexyl)-ester
- Phosphonsäuredibutylester
- Phosphonsäurediethylester
- Phosphonsäuredimethylester, bevorzugt die Phosphonsäuredioctylester.

Der aromatische Phosphonsäurediphenylester zeigt dagegen keine signifikante antiflockulierende Wirkung (Versuchsreihen 6 und 7).
a4c) Weitere Beispiele für phosphorhaltige Verbindungen mit acidem Charakter sind Phosphinsäureester.

Beispiele dafür sind Mono-Ci- bis C₁₂-Alkylphosphinate, besonders bevorzugt solche mit C₁- bis C₈-Alkylgruppen, ganz besonders bevorzugt mit C₂- bis C₈-Alkylgruppen und insbesondere solche mit C₄- bis C₈-Alkylgruppen.

Weitere Beispiele dafür sind Mono-C₆- bis C₁₂-Arylphosphinate, besonders bevorzugt solche mit C₆-Arylgruppen.

Die Alkylgruppen in Dialkylphosphinaten können dabei gleich oder verschieden sein, bevorzugt sind sie gleich.

Beispiele für C₁- bis C₁₂-Alkylgruppen sind Methyl, Ethyl, iso-Propyl, n-Propyl, n-Butyl, iso-Butyl, sek-Butyl, tert.-Butyl, n-Hexyl, n-Heptyl, n-Octyl, n-Decyl, n-Dodecyl, 2-Ethylhexyl und 2-Propylheptyl.

Die Phosphinate können am zentralen Phosphoratom einen Substituenten tragen, bei dem es sich um einen Alkyl- oder Arylrest handeln kann und der optional mit dem Sauerstoffatom verbunden sein kann und so eine Estergruppe bildet.

Beispiele dafür sind Phosphaphenanthren-Derivate wie das
- Sanko HCA = "DOPO" = 9,10-Dihydro-9-oxa-10-phosphaphenanthren-10-oxide. (CAS 35948-25-5; s. folgende Abbildung), und Derivate dieser Struktur. 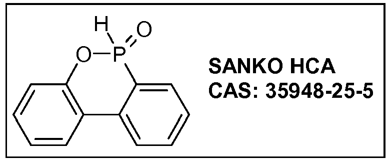 und dessen Derivate.

a4d) Ebenfalls umfasst sind Alkylderivate des Typs H₄P₂Oₙ, der Hypodiphosphonsäure, Diphosphonsäure, Hypodiphosphorsäure und Diphosphorsäure mit mindestens einer OH- oder PHaciden Gruppe.

Als Lösungsmittel für die Polyisocyanatkomponente, wie auch die Bindemittel- und ggf. weitere Komponenten einsetzbar sind solche, die keine gegenüber Isocyanatgruppen oder verkappten Isocyanatgruppen reaktiven Gruppen aufweisen und in denen die Polyisocyanate zu mindestens 10 Gew%, bevorzugt zu mindestens 25, besonders bevorzugt zu mindestens 50, ganz besonders bevorzugt zu mindestens 75, insbesondere zu mindestens 90 und speziell zu mindestens 95 Gew% löslich sind.

Beispiele für derartige Lösungsmittel sind aromatische (einschließlich alkylierter Benzole und Naphthaline) und/oder (cyclo)aliphatische Kohlenwasserstoffe und deren Gemische, chlorierte Kohlenwasserstoffe, Ketone, Ester, alkoxylierte Alkansäurealkylester, Ether, respektive Gemische der Lösungsmittel.

Als aromatische Kohlenwasserstoffgemische sind solche bevorzugt, die überwiegend aromatische C₇- bis C₁₄-Kohlenwasserstoffe umfassen und einen Siedebereich von 110 bis 300 °C umfassen können, besonders bevorzugt sind Toluol, o-, m- oder p-Xylol, Trimethylbenzolisomere, Tetramethylbenzolisomere, Ethylbenzol, Cumol, Tetrahydronaphthalin und solche enthaltende Gemische.

Beispiele dafür sind die Solvesso®-Marken der Firma ExxonMobil Chemical, besonders Solvesso® 100 (CAS-Nr. 64742-95-6, überwiegend C₉ und C₁₀-Aromaten, Siedebereich etwa 154 - 178 °C), 150 (Siedebereich etwa 182 - 207 °C) und 200 (CAS-Nr. 64742-94-5), sowie die Shellsol®-Marken der Firma Shell, Caromax® (z.B. Caromax® 18) der Firma Petrochem Carless und Hydrosol der Firma DHC (z.B. als Hydrosol® A 170). Kohlenwasserstoffgemische aus Paraffinen, Cycloparaffinen und Aromaten sind auch unter den Bezeichnungen Kristallöl (beispielsweise Kristallöl 30, Siedebereich etwa 158 - 198 °C oder Kristallöl 60: CAS-Nr. 64742-82-1), Testbenzin (beispielsweise ebenfalls CAS-Nr. 64742-82-1) oder Solventnaphtha (leicht: Siedebereich etwa 155 - 180 °C, schwer: Siedebereich etwa 225 - 300 °C) im Handel erhältlich. Der Aromatengehalt derartiger Kohlenwasserstoffgemische beträgt in der Regel mehr als 90 Gew%, bevorzugt mehr als 95, besonders bevorzugt mehr als 98 und ganz besonders bevorzugt mehr als 99 Gew%. Es kann sinnvoll sein, Kohlenwasserstoffgemische mit einem besonders verringerten Gehalt an Naphthalin einzusetzen.

(Cyclo)aliphatische Kohlenwasserstoffe sind beispielsweise Dekalin, alkyliertes Dekalin und Isomerengemische von geradlinigen oder verzweigten Alkanen und/oder Cycloalkanen.

Der Gehalt an aliphatischen Kohlenwasserstoffen beträgt in der Regel weniger als 5, bevorzugt weniger als 2,5 und besonders bevorzugt weniger als 1 Gew%.

Ester sind beispielsweise n-Butylacetat, Ethylacetat, 1-Methoxypropylacetat-2 und 2-Methoxyethylacetat.

Ether sind beispielsweise THF, Dioxan sowie die Dimethyl-, -ethyl- oder -n-butylether von Ethylenglykol, Diethylenglykol, Triethylenglykol, Propylenglykol, Dipropylenglykol oder Tripropylenglykol.

Ketone sind beispielsweise Aceton, Diethylketon, Ethylmethylketon, Isobutylmethylketon, Methylamylketon und tert.-Butylmethylketon.

Bevorzugte Lösungsmittel sind n-Butylacetat, Ethylacetat, 1-Methoxypropylacetat-2, 2-Methoxyethylacetat, sowie deren Gemische, insbesondere mit den oben aufgeführten aromatischen Kohlenwasserstoffgemischen, insbesondere Xylol und Solvesso® 100.
Derartige Gemische können im Volumenverhältnis 5:1 bis 1:5 erstellt werden, bevorzugt im Volumenverhältnis 4:1 bis 1:4, besonders bevorzugt im Volumenverhältnis 3:1 bis 1:3 und ganz besonders bevorzugt im Volumenverhältnis 2:1 bis 1:2.
Bevorzugte Beispiele sind Butylacetat/Xylol, Methoxypropylacetat/Xylol 1:1, Butylacetat/Solventnaphtha 100 1:1, Butylacetat/Solvesso® 100 1:2 und Kristallöl 30/Shellsol® A 3:1. Bevorzugt sind Butylacetat, 1-Methoxypropylacetat-2, Methylamylketon, Xylol und Solvesso® 100.

Bevorzugt werden die antiflockulierend wirkenden Additive homogen verteilt, bevorzugt unter Nutzung von Mischvorrichtungen.

Die antiflockulierend wirkenden Additive können auf vielfältige Art zugegeben werden, beispielsweise tel quel, in Lösungsmitteln und/oder anderen Additiven, insbesondere auch in anderen Lösungsmittel, in dem es sich bevorzugt gut lösen sollte, wie beispielsweise Dialkyldicarbonsäureester wie Dioctyladipinsäureester und Dinonyladipinsäureester, Phosphorsäuretrialkylestern, Phthalsäureester und kernhydrierte Phthalsäureester, wie beispielsweise Cyclohexan-1,2- dicarbonsäurediisononylester.

Die antiflockulierend wirkenden Additive können beispielsweise eingebracht werden
- zu dem reinen Polyisocyanat
- zur einem verkaufsfähigen Polyisocyanate in Lösungsmittel,
- in einer Polyisocyanatkomponente, z.B. bestehend aus Polyisocyanat, Lösungsmittel und Additiven. Solche eine verkaufsfähige Polyisocyanatkomponente kann mit einer entsprechenden Polyolkomponente ohne weitere Zugaben, ggf. nach getrennter Lagerung, durch Abmischung der beiden Komponenten zur Lackierung eingesetzt werden.

Bevorzugt sind Additive, die in flüssiger Form vorliegen, oder mit geringen und kurzzeitig wirkenden Scherkräften eingerührt werden können.
Die antiflockulierend wirkenden Additive können zum Polyisocyanat bei erhöhter Temperatur direkt nach der Destillation bei ca. 170 °C bis Umgebungstemperatur, bei festen Additiven bevorzugt bei einer Temperatur über ihrem Schmelzpunkt, oder auch fest bei dann erhöhtem Aufwand zur Homogenisierung zugegeben werden.
In einer speziellen Form werden sie batchweise, ggf. in Lösungsmittel, in weniger als einer Stunde zwischen Raumtemperatur und 70 °C in einem Rührkessel mit dem Polyisocyanat vermischt.
In einer anderen speziellen Form werden sie, ggf. in Lösungsmittel, zwischen Raumtemperatur und 70 °C kontinuierlich in einem statischen Mischer zugegeben.

Ein Vorlösen in Lösungsmittel kann insbesondere bei festen antiflockulierend wirkenden Additiven wie den mehrkernigen Phosphiten a2) oder Phosphoniten a3) mit z.T. hohen Schmelzpunkten und schlechten Löslichkeiten in Polyisocyanat und Lösungsmittel sinnvoll sein.

Eine weitere Anwendungsform ist das Ansetzen einer Stammlösung, z.B. in Lösungsmittel, Additivgemischen und / oder Polyisocyanat.

Die Mengen in denen die Antiflockulanzien zugegeben werden, sind in den unten genannten Beispielen üblicherweise signifikant unterstöchiometrisch in Bezug auf die Gesamtwassermenge in der Mischung. Dies soll jedoch die zugegebenen Mengen Additive im Rahmen der Ansprüche nicht auf einen stöchiometrischen Unterschuss beschränken.

In einer bevorzugten Form ist es vorteilhaft aus den oben genannten und weiteren Gründen, die minimal notwendige Menge Additiv zum Polyisocyanat zu geben mit der eine Flockulation, z.B. für ein halbes Jahr ausreichend unterbunden wird. Weitere Gründe für die Festlegung einer minimal notwendigen Menge ist, dass manche der Additive weitere funktionelle Wirkung in Polyurethansystemen haben können. Aromatische Sulfonsäuren können z.B. als Urethanisierungkatalysator wirken. Dialkylphosphorsäuren können in Wechselwirkung mit Dibutylzinndilaurat abhängig von der Menge zu Niederschlägen führen. Säuren können in Wechselwirkung mit basischen Additiven wie UV-Stabilisatoren oder Aminkatalysatoren treten. Von daher ist es vorteilhaft die Additivmenge möglichst gering zu halten, und ggf. abhängig von sonstigen Additiven selektiv auszuwählen.

Die antiflockulierende Wirkung nimmt nicht immer mit der Additiv-Menge zu. Die optimale Menge muss daher ggf. abhängig vom eingesetzten Polyisocyanat und anderen Komponenten bestimmt werden.
Im Allgemeinen werden die erfindungsgemäßen Verbindungen dem Polyisocyanat zur Verminderung der Flockulation in den folgenden Mengen zugesetzt:
Verbindungen a1a) werden in der Regel in Mengen bezogen auf das Polyisocyanat von 1 bis 600 Gew.ppm, bevorzugt 2 bis 100, besonders bevorzugt 5 bis 50 Gew.ppm zugesetzt. Verbindungen a1b), a2), a3) werden in der Regel in Mengen bezogen auf das Polyisocyanat von 20 bis 3000 Gew.ppm, bevorzugt 50-800 Gew.ppm, besonders bevorzugt 100-600 Gew.ppm zugesetzt.
Verbindungen a4a) werden in der Regel in Mengen bezogen auf das Polyisocyanat von 5 bis 1000 Gew.ppm, bevorzugt 10 bis 600, besonders bevorzugt 20 bis 200, ganz besonders bevorzugt 20 bis 100 Gew.ppm zugesetzt.
Verbindungen a4b), a4c), a4d) werden in der Regel in Mengen bezogen auf das Polyisocyanat von 5 bis 1000 Gew.ppm, bevorzugt 10 bis 600, besonders bevorzugt 20 bis 300, ganz besonders bevorzugt 30 bis 200 Gew.ppm zugesetzt.

Die Wahl der Additive kann abhängig vom eingesetzten Lösungsmittel oder Lösungsmittelgemisch sein.

Häufig gilt, dass die Flockulationsneigung mit zunehmender Verdünnung durch das Lösungsmittel zunimmt, insbesondere bei Lösungsmittelgehältern über 50 %, insbesondere bei über 60 %.

Die Polyisocyanatkomponente enthaltend ein Additiv mit antiflockulierender Wirkung kann, ggf. nach Lagerung, mit mindestens einer Bindemittelkomponente und ggf., weiteren Komponenten z.B. zu Lacken oder Klebstoffen umgesetzt werden.

Bei den Bindemitteln kann es sich beispielsweise um Polyacrylatpolyole, Polyesterpolyole, Polyetherpolyole, Polyurethanpolyole; Polyharnstoffpolyole; Polyesterpolyacrylatpolyole; Polyesterpolyurethanpolyole; Polyurethanpolyacrylatpolyole, Polyurethanmodifizierte Alkydharze; Fettsäuremodifizierte Polyesterpolyurethanpolyole, Kopolymerisate mit Allylethern, Propfpolymerisate aus den genannten Stoffgruppen mit z.B. unterschiedlichen Glasübergangstemperaturen, sowie Mischungen der genannten Bindemittel handeln. Bevorzugt sind Polyacrylatpolyole, Polyesterpolyole und Polyurethanpolyole.

Bevorzugte OH-Zahlen, gemessen gemäß DIN 53240-2 (potentiometrisch), sind 40-350 mg KOH/g Festharz für Polyester, bevorzugt 80-180 mg KOH/g Festharz, und 15-250 mg KOH/g Festharz für Polyacrylatole, bevorzugt 80-160 mg KOH/g.

Zusätzlich können die Bindemittel eine Säurezahl gemäß DIN EN ISO 3682 (potentiometrisch) bis 200 mg KOH/g, bevorzugt bis 150 und besonders bevorzugt bis 100 mg KOH/g aufweisen.

Besonders bevorzugte Bindemittel sind Polyacrylatpolyole und Polyesterole.

Polyacrylatpolyole weisen bevorzugt ein Molekulargewicht Mₙ von mindestens 500, besonders bevorzugt mindestens 1200 g/mol auf. Das Molekulargewicht Mₙ kann prinzipiell nach oben unbegrenzt sein, bevorzugt bis zu 50.000, besonders bevorzugt bis zu 20.000 g/mol, ganz besonders bevorzugt bis zu 10.000 g/mol und insbesondere bis zu 5.000 g/mol betragen.

Die hydroxyfunktionellen Monomere (siehe unten) werden in solchen Mengen bei der Kopolymerisation mit verwendet, dass die obengenannten Hydroxylzahlen der Polymere resultieren, die im allgemeinen einem Hydroxygruppengehalt der Polymere von 0,5 bis 8, vorzugsweise 1 bis 5 Gew.-% entsprechen.

Dabei handelt es sich um hydroxygruppenhaltige Kopolymere aus mindestens einem hydroxygruppenhaltigen (Meth)acrylat mit mindestens einem weiteren polymerisationsfähigen Komonomer ausgewählt aus der Gruppe bestehend aus (Meth)acrylsäurealkylestern, Vinylaromaten, α, β-ungesättigten Carbonsäuren und anderen Monomeren.

Als (Meth)acrylsäurealkylestern genannt seien z.B. C₁-C₂₀-Alkyl(meth)acrylate, Vinylaromaten sind solche mit bis zu 20 C-Atomen, α,β-ungesättigte Carbonsäuren umfassen auch deren Anhydride und andere Monomere sind beispielsweise Vinylester von bis zu 20 C-Atomen enthaltenden Carbonsäuren, ethylenisch ungesättigte Nitrile, Vinylether von 1 bis 10 C-Atome enthaltenden Alkoholen und, weniger bevorzugt, aliphatische Kohlenwasserstoffe mit 2 bis 8 C-Atomen und 1 oder 2 Doppelbindungen.

Als (Meth)acrylsäurealkylester bevorzugt sind solche mit einem C₁-C₁₀-Alkylrest, wie Methylmethacrylat, Methylacrylat, n-Butylacrylat, Ethylacrylat und 2-Ethylhexylacrylat.

Insbesondere sind auch Mischungen der (Meth)acrylsäurealkylester geeignet.

Vinylester von Carbonsäuren mit 1 bis 20 C-Atomen sind z.B. Vinyllaurat, Vinylstearat, Vinylpropionat und Vinylacetat.

α, β-Ungesättigte Carbonsäuren und deren Anhydride können beispielsweise sein Acrylsäure, Methacrylsäure, Fumarsäure, Crotonsäure, Itaconsäure, Maleinsäure oder Maleinsäureanhydrid, bevorzugt Acrylsäure.

Als hydroxyfunktionelle Monomere seien Monoester von α,β-ungesättigten Carbonsäuren, wie Acrylsäure, Methacrylsäure (in dieser Schrift kurz als "(Meth)acrylsäure" bezeichnet), mit Di- oder Polyolen erwähnt, die vorzugsweise 2 bis 20 C-Atome und wenigstens zwei Hydroxygruppen aufweisen, wie Ethylenglykol, Diethylenglykol, Triethylenglykol, 1,2-Propylenglykol, 1,3-Propylenglykol, 1,1-Dimethyl-1,2-Ethandiol, Dipropylenglykol, Triethylenglykol, Tetraethylenglykol, Pentaethylenglykol, Tripropylenglykol, 1,4-Butandiol, 1,5-Pentandiol, Neopentylglykol, Hydroxypivalinsäureneopentylglykolester, 2-Ethyl-1,3-Propandiol, 2-Methyl-1,3-Propandiol, 2-Butyl-2-ethyl-1,3-Propandiol, 1,6-Hexandiol, 2-Methyl-1,5-pentandiol, 2-Ethyl-1,4-butandiol, 2-Ethyl-1,3-Hexandiol, 2,4-Diethyl-oktan-1,3-diol, 2,2-Bis(4-hydroxycyclohexyl)propan, 1,1-, 1,2-, 1,3- und 1,4-Bis(hydroxymethyl)-cyclohexan, 1,2-, 1,3- oder 1,4-Cyclohexandiol, Glycerin, Trimethylolethan, Trimethylolpropan, Trimethylolbutan, Pentaerythrit, Ditrimethylolpropan, Dipentaerythrit, Sorbit, Mannit, Diglycerol, Threit, Erythrit, Adonit (Ribit), Arabit (Lyxit), Xylit, Dulcit (Galactit), Maltit, Isomalt, Poly-THF mit einem Molekulargewicht zwischen 162 und 4500, bevorzugt 250 bis 2000, Poly-1,3-propandiol oder Polypropylenglykol mit einem Molekulargewicht zwischen 134 und 2000 oder Polyethylenglykol mit einem Molekulargewicht zwischen 238 und 2000 sein.

Bevorzugt sind 2-Hydroxyethylacrylat, 2-Hydroxyethylmethacrylat, 2- oder 3-Hydroxypropylacrylat, 1,4-Butandiolmonoacrylat oder 3-(Acryloyloxy)-2-hydroxypropylacrylat und besonders bevorzugt 2-Hydroxyethylacrylat und/oder 2-Hydroxyethylmethacrylat.

Als vinylaromatische Verbindungen kommen z.B. Vinyltoluol, α-Butylstyrol, α-Methylstyrol, 4-n-Butylstyrol, 4-n-Decylstyrol und vorzugsweise Styrol in Betracht.

Beispiele für Nitrile sind Acrylnitril und Methacrylnitril.

Geeignete Vinylether sind z.B. Vinylmethylether, Vinylisobutylether, Vinylhexylether und Vinyloctylether.

Als nicht aromatische Kohlenwasserstoffe mit 2 bis 8 C-Atomen und einer oder zwei olefinischen Doppelbindungen seien Butadien, Isopren, sowie Ethylen, Propylen und Isobutylen genannt.

Weiterhin sind N-Vinylformamid, N-Vinylpyrrolidon sowie N-Vinylcaprolactam einsetzbar, weiterhin, ethylenisch ungesättigte Säuren, insbesondere Carbonsäuren, Säureanhydride oder Säureamide, sowie Vinylimidazol. Auch Epoxidgruppen aufweisende Komonomere wie z.B. Glycidylacrylat oder -methacrylat oder Monomere wie N-Methoxymethylacrylamid oder- methacrylamid können in geringen Mengen mit verwendet werden.

Bevorzugt sind Ester der Acrylsäure bzw. der Methacrylsäure mit 1 bis 18, vorzugsweise 1 bis 8 Kohlenstoffatomen im Alkoholrest wie z.B. Methylacrylat. Ethylacrylat, Iso-propylacrylat, n-Propylacrylat, n-Butylacrylat, 2-Ethylhexylacrylat, n-Stearylacrylat, die diesen Acrylaten entsprechenden Methacrylate, Styrol, alkylsubstituierte Styrole, Acrylnitril, Methacrylnitril, Vinylacetat oder Vinylstearat bzw. beliebige Gemische derartiger Monomere.

Die Hydroxygruppen tragenden Monomere werden in die Kopolymerisation der Hydroxygruppen tragenden (Meth)Acrylate im Gemisch mit anderen polymerisierbaren, bevorzugt radikalisch polymerisierbaren Monomeren, eingesetzt, bevorzugt solche, welche zu mehr als 50 Gew% aus C₁-C₂₀-, bevorzugt C₁- bis C₄-Alkyl(meth)acrylat, (Meth)acrylsäure, Vinylaromaten mit bis zu 20 C-Atomen, Vinylestern von bis zu 20 C-Atomen enthaltenden Carbonsäuren, Vinylhalogeniden, nicht aromatischen Kohlenwasserstoffen mit 4 bis 8 C-Atomen und 1 oder 2 Doppelbindungen, ungesättigten Nitrilen und deren Mischungen bestehen. Besonders bevorzugt sind die Polymere, die neben den Hydroxygruppen tragenden Monomeren zu mehr als 60 Gew% aus C₁-C₁₀-Alkyl(meth)acrylaten, Styrol und dessen Derivaten oder deren Mischungen bestehen.

Die Herstellung der Polymerisate kann durch Polymerisation nach üblichen Verfahren durchgeführt werden. Vorzugsweise erfolgt die Herstellung der Polymerisate in einer Emulsionspolymerisation oder in organischer Lösung. Möglich sind kontinuierliche oder diskontinuierliche Polymerisationsverfahren. Von den diskontinuierlichen Verfahren sind das Batch- und das Zulaufverfahren zu nennen, wobei letzteres bevorzugt ist. Bei dem Zulaufverfahren wird das Lösungsmittel allein oder mit einem Teil des Monomergemisches vorgelegt, auf die Polymerisationstemperatur erwärmt, die Polymerisation im Fall einer Monomervorlage radikalisch gestartet und das restliche Monomergemisch zusammen mit einem Initiatorgemisch im Verlauf von 1 bis 10 Stunden, vorzugsweise 3 bis 6 Stunden, zudosiert. Optional wird anschließend noch nachaktiviert, um die Polymerisation bis zu einem Umsatz von mindestens 99 % durchzuführen.

Als Lösungsmittel kommen beispielsweise Aromaten, wie Solventnaphtha, Benzol, Toluol, Xylol, Chlorbenzol, Ester wie Ethylacetat, Butylacetat, Methylglykolacetat,
Ethylglykolacetat, Methoxypropylacetat, Ether wie Butylglykol, Tetrahydrofuran, Dioxan, Ethylglykolether, Ketone wie Aceton, Methylethylketon, halogenhaltige Lösemittel wie Methylenchlorid oder Trichlormonofluorethan in Betracht.

Weitere Bindemittel sind z.B. Polyesterpolyole, wie sie durch Kondensation von Polycarbonsäuren, insbesondere Dicarbonsäuren mit Polyolen, insbesondere Diolen erhältlich sind. Um eine für die Polymerisation angemessene Funktionalität des Polyesterpolyols zu gewährleisten werden partiell auch Triole, Tetrole etc. wie auch Trisäuren, etc. eingesetzt.

Polyesterpolyole, sind z.B. aus Ullmanns Enzyklopädie der technischen Chemie, 4. Auflage, Band 19, S. 62 bis 65 bekannt. Bevorzugt werden Polyesterpolyole eingesetzt, die durch Umsetzung von zweiwertigen Alkoholen mit zweiwertigen Carbonsäuren erhalten werden. Anstelle der freien Polycarbonsäuren können auch die entsprechenden Polycarbonsäureanhydride oder entsprechende Polycarbonsäureester von niederen Alkoholen oder deren Gemische zur Herstellung der Polyesterpolyole verwendet werden. Die Polycarbonsäuren können aliphatisch, cycloaliphatisch, aromatisch oder heterocyclisch sein und optional, z.B. durch Halogenatome, substituiert und/oder ungesättigt sein. Als Beispiele hierfür seien genannt:
Oxalsäure, Maleinsäure, Fumarsäure, Bernsteinsäure, Glutarsäure, Adipinsäure, Sebacinsäure, Dodekandisäure, o-Phthalsäure, Isophthalsäure, Terephthalsäure, Trimellithsäure, Azelainsäure, 1,4-Cyclohexandicarbonsäure oder Tetrahydrophthalsäure, Korksäure, Azelainsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäureanhydrid, dimere Fettsäuren, deren Isomere und Hydrierungsprodukte sowie veresterbare Derivate, wie Anhydride oder Dialkylester, beispielsweise C₁-C₄-Alkylester, bevorzugt Methyl-, Ethyl- oder n-Butylester, der genannten Säuren eingesetzt werden. Bevorzugt sind Dicarbonsäuren der allgemeinen Formel HOOC-(CH₂)_{y}-COOH, wobei y eine Zahl von 1 bis 20, bevorzugt eine gerade Zahl von 2 bis 20 ist, besonders bevorzugt Bernsteinsäure, Adipinsäure, Sebacinsäure und Dodecandicarbonsäure.

Als mehrwertige Alkohole kommen zur Herstellung der Polyesterole in Betracht 1,2-Propandiol, Ethylenglykol, 2,2-Dimethyl-1,2-Ethandiol, 1,3-Propandiol, 1,2-Butandiol, 1,3-Butandiol, 1,4-Butandiol, 3-Methylpentan-1,5-diol, 2-Ethylhexan-1,3-diol, 2,4-Diethyloctan-1,3-diol, 1,6-Hexandiol, Poly-THF mit einer Molmasse zwischen 162 und 4500, bevorzugt 250 bis 2000, Poly-1,3-propandiol mit einer Molmasse zwischen 134 und 1178, Poly-1,2-propandiol mit einer Molmasse zwischen 134 und 898, Polyethylenglykol mit einer Molmasse zwischen 106 und 458, Neopentylglykol, Hydroxypivalinsäureneopentylglykolester, 2-Ethyl-1,3-Propandiol, 2-Methyl-1,3-Propandiol, 2,2-Bis(4-hydroxycyclohexyl)propan, 1,1-, 1,2-, 1,3- und 1,4-Cyclohexandimethanol, 1,2-, 1,3- oder 1,4-Cyclohexandiol, Trimethylolbutan, Trimethylolpropan, Trimethylolethan, Neopentylglykol, Pentaerythrit, Glycerin, Ditrimethylolpropan, Dipentaerythrit, Sorbit, Mannit, Diglycerol, Threit, Erythrit, Adonit (Ribit), Arabit (Lyxit), Xylit, Dulcit (Galactit), Maltit oder Isomalt, die optional wie oben beschrieben alkoxiliert sein können.

Bevorzugt sind Alkohole der allgemeinen Formel HO-(CH₂)ₓ-OH, wobei x eine Zahl von 1 bis 20, bevorzugt eine gerade Zahl von 2 bis 20 ist. Bevorzugt sind Ethylenglykol, Butan-1,4-diol, Hexan-1,6-diol, Octan-1,8-diol und Dodecan-1,12-diol. Weiterhin bevorzugt ist Neopentylglykol.

Ferner kommen auch Polycarbonat-Diole in Betracht, wie sie z.B. durch Umsetzung von Phosgen mit einem Überschuss von den als Aufbaukomponenten für die Polyesterpolyole genannten niedermolekularen Alkohole erhalten werden können.

Geeignet sind auch Polyesterdiole auf Lacton-Basis, wobei es sich um Homo- oder Mischpolymerisate von Lactonen, bevorzugt um endständige Hydroxygruppen aufweisende Anlagerungsprodukte von Lactonen an geeignete difunktionelle Startermoleküle handelt. Als Lactone kommen bevorzugt solche in Betracht, die sich von Verbindungen der allgemeinen Formel HO-(CH₂)_{z}-COOH ableiten, wobei z eine Zahl von 1 bis 20 ist und ein H-Atom einer Methyleneinheit auch durch einen C₁- bis C₄-Alkylrest substituiert sein kann. Beispiele sind ε-Caprolacton, β-Propiolacton, gamma-Butyrolacton und/oder Methyl-ε-caprolacton, 4-Hydroxybenzoesäure, 6-Hydroxy-2-naphthalinsäure oder Pivalolacton sowie deren Gemische. Geeignete Starterkomponenten sind z.B. die vorstehend als Aufbaukomponente für die Polyesterpolyole genannten niedermolekularen zweiwertigen Alkohole. Die entsprechenden Polymerisate des ε-Caprolactons sind besonders bevorzugt. Auch niedere Polyesterdiole oder Polyetherdiole können als Starter zur Herstellung der Lacton-Polymerisate eingesetzt sein. Anstelle der Polymerisate von Lactonen können auch die entsprechenden, chemisch äquivalenten Polykondensate der den Lactonen entsprechenden Hydroxycarbonsäuren, eingesetzt werden.

In Polyurethanlacken sind Molmassen Mₙ der Polyester von 800 - 4000 g/mol üblich, wobei die hier verwendeten Polyester nicht darauf beschränkt sind.

Weiterhin sind als Bindemittel auch Polyetherole geeignet, die durch Addition von Ethylenoxid, Propylenoxid und/oder Butylenoxid, bevorzugt Ethylenoxid und/oder Propylenoxid und besonders bevorzugt Ethylenoxid an H-aktive Komponenten hergestellt werden. Ebenso sind Polykondensate aus Butandiol geeignet. In Polyurethanlacken sind Molmassen der Polyether von 500-2000 g/mol üblich, wobei die hier verwendeten Polyether nicht darauf beschränkt sind.

Die Polymeren können zumindest teilweise durch sogenannte Reaktivverdünner ersetzt werden. Dabei kann es sich um blockierte sekundäre oder primäre Amine (Aldimine und Ketime) oder um Verbindungen mit sterisch gehinderten und / oder elektronenarmen sekundären Aminogruppen handeln, beispielsweise Asparaginsäureester gemäß EP 403921 oder WO 2007/39133.

Des Weiteren kann optional ein Urethanisierungskatalysator in der Beschichtungsmasse anwesend sein. Dabei kann es sich zum Beispiel um ein Amin oder eine metallorganische Verbindung halten.

Amine sind beispielsweise tertiäre aliphatische, cycloaliphatische oder aromatische Amine. Beispiele dafür sind Triethylamin, Tri-n-butylamin, N-Methylmorpholin, N-Methyl piperidin, Pyrrolidin, Chinuclidin oder 1,4-Di-aza-bicyclo-[2.2.2]-octan

Metallorganische Verbindung sind beispielsweise, wie beispielsweise Zinn-(IV)- und Zinn-(II)-salze von organischen Carbonsäuren, z.B. Zinn-(II)-diacetat, Zinn-(II)-dioctoat, Zinn-(II)-bis(ethylhexanoat) und Zinn-(II)-dilaurat. Zudem können Zink-(II)-Salze eingesetzt werden, wie beispielsweise Zink-(II)-dioctoat, -di-neo-octanoat, -diacetat, oder -oxalat. Auch Metallkomplexe wie Acetylacetonate des Eisens, Titans, Aluminiums, Zirkons, Mangans, Nickels, Zinks und Cobalts sind möglich. Weitere Metallkatalysatoren werden von Blank et al. in Progress in Organic Coatings, 1999, Vol. 35, Seiten 19-29 beschrieben.

Dialkylzinn-(IV)-salze von organischen Carbonsäuren sind z.B. Dimethylzinn-diacetat, Dibutylzinn-diacetat, Dibutylzinn-dibutyrat, Dibutylzinn-bis(2-ethylhexanoat), Dibutylzinn-dilaurat, Dibutylzinn-maleat, Dioctylzinn-dilaurat und Dioctylzinn-diacetat. Bevorzugt sind Dibutylzinndiacetat und Dibutylzinn-dilaurat. Zinnsalze sind aus toxikologischen Gründen weniger bevorzugt, werden aber in der Praxis immer noch häufig eingesetzt.

Weitere bevorzugte Lewis-saure organische Metallverbindungen sind Zink-(II)-dioctoat, Zirkonacetylacetonat und Zirkon-2,2,6,6-tetramethyl-3,5-heptandionat.

Auch Bismut- und Kobalt-Katalysatoren, Cer-Salze wie Ceroctoate, und Cäsiumsalze können als Katalysatoren eingesetzt werden.
Bismut-Katalysatoren sind insbesondere Bismut-carboxylate, insbesondere Bismut-octoate,-ethylhexanoate, -neodecanoate, oder -pivalate; beispielsweise K-KAT 348 und XK-601 von King Industries, TIB KAT 716, 716LA, 716XLA, 718, 720, 789 von TIB Chemicals und solchen von Shepherd Lausanne, sowie Katalysator-Mischungen von z.B. Bismut- und Zink-Organylen.

Als Cäsiumsalze kommen dabei solche Verbindungen in Betracht, in denen folgende Anionen eingesetzt werden: F⁻, Cl⁻, ClO⁻, ClO₃⁻, ClO₄⁻, Br⁻, J⁻, JO₃⁻, CN⁻, OCN⁻, NO₂⁻, NO₃⁻, HCO₃⁻, CO₃²⁻, S²⁻, SH⁻, HSO₃⁻, SO₃²⁻, HSO₄⁻, SO₄²⁻, S₂O₂²⁻, S₂O₄²⁻, S₂O₅²⁻, S₂O₆²⁻, S₂O₇²⁻, S₂O₈²⁻, H₂PO₂⁻, H₂PO₄⁻, HPO₄²⁻, PO₄³⁻, P₂O₇⁴⁻, (OCₙH₂ₙ₊₁)⁻, (CₙH₂ₙ₋₁O₂)⁻, (CₙH₂ₙ₋₃O₂)⁻ sowie (Cₙ₊₁H₂ₙ₋₂O₄)²⁻, wobei n für die Zahlen 1 bis 20 steht. Bevorzugt sind dabei Cäsiumcarboxylate, bei denen das Anion den Formeln
(CₙH₂ₙ₋₁O₂)⁻ sowie (Cₙ₊₁H₂ₙ₋₂O₄)²⁻ mit n gleich 1 bis 20, gehorcht. Besonders bevorzugte Cäsiumsalze weisen als Anionen Monocarboxylate der allgemeinen Formel
(CₙH₂ₙ₋₁O₂)⁻ auf, wobei n für die Zahlen 1 bis 20 steht. Hierbei sind insbesondere zu erwähnen Format, Acetat, Propionat, Hexanoat und 2-Ethylhexanoat.

Als weitere lacktypische Komponenten und/oder Additive können beispielsweise verwendet werden: Stabilisatoren, UV-Stabilisatoren wie UV-Absorber und geeignete Radikalfängern (insbesondere HALS-Verbindungen, Hindered Amin Light Stabilizer), Aktivatoren (Beschleuniger), Trockenmittel, Füllmittel, Pigmente, Farbstoffe, antistatische Agenzien, Flammschutzmittel, Verdicker, thixotrope Agenzien, oberflächenaktive Agenzien, Viskositätsmodifikatoren, Plastifizierer oder Chelatbildner. Bevorzugt sind UV-Stabilisatoren.

Stabilisatoren sind mindestens eine stabilisierend wirkende Verbindung, wobei mit "stabilisierend" die Eigenschaft bezeichnet ist, die Entwicklung einer Farbzahl und/oder der Viskosität des Polyisocyanats im Verlauf der Lagerung über einen gewissen Zeitraum gegenüber solchen entsprechenden Mischungen zu verringern, die keine stabilisierend wirkenden Verbindungen enthalten.
Die Stabilisierung kann sich sowohl auf das Polyisocyanat alleine beziehen, wie auch auf Vormischungen der Polyisocyanate mit weiteren lacktypischen Komponenten und/oder Additiven, optional unter Zugabe weiterer Komponenten. Dies beinhaltet in einer besonderen Ausführung die Lagerung einer dieser Komponenten vor der eigentlichen Lackapplizierung.

Bevorzugt sind diese stabilisierend wirkenden Verbindungen ausgewählt aus der Gruppe bestehend aus primären Antioxidantien (Radikalfänger), sekundären Antioxidantien (Verbindungen, die die Radikalbildung verhindern, insbesondere indem sie Peroxide abfangen und/oder zersetzen) und sauren Stabilisatoren (Bronsted-Säuren).

Bei den primären Antioxidantien handelt es sich bevorzugt um sterisch gehinderte Phenole. Derartige sterisch gehinderte Phenole sind beispielsweise beschrieben in WO 2008/116894, bevorzugt die dort von Seite 14, Zeile 10 bis Seite 16, Zeile 10 beschriebenen Verbindungen, was hiermit per Bezugnahme Bestandteil der vorliegenden Offenbarung sei.

Dabei handelt es sich bevorzugt um solche Phenole, die an dem aromatischen Ring genau eine phenolische Hydroxygruppe aufweisen und besonders bevorzugt um solche, die in den ortho-Positionen, ganz besonders bevorzugt in ortho- und para-Position zur phenolischen Hydroxygruppe einen beliebigen Substituenten, bevorzugt eine Alkylgruppe aufweisen, insbesondere um Alkyl-3-(3,5-di-tert-butyl-4-hydroxyphenyl)-propionate, respektive um substituierte AlkylDerivate solcher Verbindungen.
In Verbindung mit erfindungsgemäßen Additiven sind sie Teil der Erfindung, bevorzugt 2,6-Di-tert.-butyl-4-methylphenol (BHT); Isooctyl-3-(3,5-di-tert-butyl-4-hydroxyphenyl)-propionat (z.B. Irganox® 1135), Octadecyl-3-(3,5-di-tert-butyl-4-hydroxyphenyl)-propionat (z.B. Irganox® 1076).

Solche Phenole können auch Bestandteile eines polyphenolischen Systems mit mehren Phenol-Gruppen sein Pentaerythrit-tetrakis(3-(3,5-di-tert-butyl-4-hydroxyphenyl)propionat (z.B. Irganox® 1010); Ethylen-bis(oxyethylen)-bis-(3-(5-tert.-butyl-4-hydroxy-m-tolyl)-propionat) (z.B. Irganox 245); 3,3',3'',5,5',5''-Hexa-tert-butyl-a,a',a''-(mesitylen-2,4,6-triyl)tri-p-kresol (z.B. Irganox® 1330); 1,3,5-Tris(3,5-di-tert-butyl-4-hydroxybenzyl)-1,3,5-triazin-2,4,6(1H,3H,5H)-trion (z.B. Irganox® 3114), jeweils Produkte der Ciba Spezialitätenchemie, jetzt BASF SE.

Die sekundären Antioxidantien sind bevorzugt ausgewählt aus der Gruppe bestehend aus Phosphoniten, Phosphonaten und Thioethern, bevorzugt aus Phosphoniten oder Phosphonaten.

Bevorzugte Phosphonite sind beschrieben in WO 2008/116894, dort besonders von Seite 11, Zeile 8 bis Seite 14, Zeile 8, was hiermit per Bezugnahme Bestandteil der vorliegenden Offenbarung sei.

Bevorzugte Phosphonate sind beschrieben in WO 2008/116895, dort besonders von Seite 10, Zeile 38 bis Seite 12, Zeile 41, was hiermit per Bezugnahme Bestandteil der vorliegenden Offenbarung sei.

Diese letztgenannten Phosphonite und Phosphonate unterscheiden sich von den erfindungsgemäß eingesetzten Verbindungen a3) bzw. a4) nicht zuletzt dadurch, dass sie in der Beschichtungsmasse einen anderen Zweck erfüllen, nämlich eine antioxidative Wirkung, verglichen mit der Wirkung als Antiflockulanz gemäß der vorliegenden Erfindung.

Bevorzugte Thioether sind beschrieben in WO 2008/116893, dort besonders von Seite 11, Zeile 1 bis Seite 15, Zeile 37, was hiermit per Bezugnahme Bestandteil der vorliegenden Offenbarung sei.

Bei den sauren Stabilisatoren handelt es sich um Bronsted-Säuren, wie sie beschrieben sind in WO 2008/116894, dort besonders von Seite 17, Zeile 34 bis Seite 18, Zeile 23, was hiermit per Bezugnahme Bestandteil der vorliegenden Offenbarung sei.

Geeignete UV-Absorber umfassen Oxanilide, Triazine und Benzotriazole (letztere erhältlich z.B. als Tinuvin® -Marken der BASF SE) und Benzophenone (z.B. Chimassorb® 81 der BASF SE). Bevorzugt sind z.B. 95 % Benzolpropansäure, 3-(2H-Benzotriazol-2-yl)-5-(1,1-dimethylethyl)-4-hydroxy-, C7-9-verzweigte und lineare Alkylester; 5 % 1-Methoxy-2-propylacetat (z.B. Tinuvin® 384) und α-[3-[3-(2H-Benzotriazol-2-yl)-5-(1,1,-dimethylethyl)-4-hydroxyphenyl]-1-oxopropyl]-ω-hydroxypoly(oxo-1,2-ethanediyl) (z.B. Tinuvin® 1130), jeweils Produkte z.B. der BASF SE. DL-Alpha-Tocopherol, Tocopherol, Zimtsäurederivate und Cyanoacrylate können ebenfalls zu diesem Zweck eingesetzt werden.

Diese können allein oder zusammen mit geeigneten Radikalfängern, beispielsweise sterisch gehinderten Aminen (oft auch als HALS -oder HAS-Verbindungen bezeichnet; Hindered Amine (Light) Stabilizer) wie 2,2,6,6-Tetramethylpiperidin, 2,6-Di-tert.-butylpiperidin oder deren Derivaten, z. B. Bis-(2,2,6,6-tetra-methyl-4-piperidyl)sebacinat, eingesetzt werden. Diese sind z.B. erhältlich als Tinuvin®- und Chimassorb®-Marken der BASF SE. Bevorzugt im gemeinsamen Einsatz mit Lewis-Säuren sind jedoch solche gehinderten Amine, die N-alkyliert sind, beispielsweise Bis (1,2,2,6,6-pentamethyl-4-piperidinyl)-[[3,5-bis(1,1-dimethylethyl)-4-hydroxyphenyl]-methyl]butylmalonat (z.B. Tinuvin® 144 der BASF SE); eine Mischung aus Bis(1,2,2,6,6-Pentamethyl-4-Piperidinyl)sebacat und Methyl(1,2,2,6,6-Pentamethyl-4-Piperidinyl)sebacat (z.B. Tinuvin® 292 der BASF SE); oder die N-(O-alkyliert) sind, wie z.B. Dekandisäure-bis-(2,2,6,6-tetramethyl-1-(octyloxy)-4-piperidinyl)-ester, Reaktionsprodukte mit 1,1-Dimethylethylhydroperoxyd und Oktan (z.B. Tinuvin® 123 der BASF SE), und speziell dem HALS-Triazin 2,4-Bis[N-butyl-N-(1-cyclohexyloxy-2,2,6,6,-tetramethylpiperidin-4-yl)amino]-6-(2-hydroxyethylamin)-1,3,5-triazin (z.B. Tinuvin® 152 der BASF SE). Säureunempfindliche Additive wie das Tinuvin® 152 sind dabei vorteilhaft.

UV-Stabilisatoren werden üblicherweise in Mengen von 0,1 bis 5,0 Gew.-%, bezogen auf die in der Zubereitung enthaltenen festen Komponenten, eingesetzt.

Als Verdicker kommen neben radikalisch (ko)polymerisierten (Ko)Polymeren, übliche organische und anorganische Verdicker wie Hydroxymethylcellulose oder Bentonit in Betracht.

Als Chelatbildner können z.B. Ethylendiaminessigsäure und deren Salze sowie β-Diketone verwendet werden.

Pigmente im eigentlichen Sinne sind gemäß CD Römpp Chemie Lexikon - Version 1.0, Stuttgart/New York: Georg Thieme Verlag 1995 unter Verweis auf DIN 55943 partikelförmige "im Anwendungsmedium praktisch unlösliche, anorganische oder organische, bunte oder unbunte Farbmittel".

Praktisch unlöslich bedeutet dabei eine Löslichkeit bei 25 °C unter 1 g / 1000 g Anwendungsmedium, bevorzugt unter 0,5, besonders bevorzugt unter 0,25, ganz besonders bevorzugt unter 0,1 und insbesondere unter 0,05 g / 1000 g Anwendungsmedium.

Beispiele für Pigmente im eigentlichen Sinne umfassen beliebige Systeme von Absorptions- und/oder Effektpigmenten, bevorzugt Absorptionspigmente. Anzahl und Auswahl der Pigmentkomponenten sind dabei keinerlei Beschränkungen unterworfen. Sie können den jeweiligen Erfordernissen, beispielsweise dem gewünschten Farbeindruck, beliebig angepasst werden, beispielsweise wie in Schritt a) beschrieben. Beispielsweise können alle Pigmentkomponenten eines standardisierten Mischlacksystems zugrunde liegen.

Unter Effektpigmenten sind alle Pigmente zu verstehen, die einen plättchenförmigen Aufbau zeigen und einer Oberflächenbeschichtung spezielle dekorative Farbeffekte verleihen. Bei den Effektpigmenten handelt es sich beispielsweise um alle in der Fahrzeug- und Industrielackierung üblicherweise einsetzbaren effektgebenden Pigmente. Beispiele für derartige Effektpigmente sind reine Metallpigmente; wie z.B. Aluminium-, Eisen- oder Kupferpigmente; Interferenzpigmente, wie z.B. titandioxidbeschichteter Glimmer, eisenoxidbeschichteter Glimmer, mischoxidbeschichteter Glimmer (z.B. mit Titandioxid und Fe₂O₃ oder Titandioxid und Cr₂O₃), metalloxidbeschichtetes Aluminium, oder Flüssigkristallpigmente.

Bei den farbgebenden Absorptionspigmenten handelt es sich beispielsweise um übliche in der Lackindustrie einsetzbare organische oder anorganische Absorptionspigmente. Beispiele für organische Absorptionspigmente sind Azopigmente, Phthalocyanin-, Chinacridon- und Pyrrolopyrrolpigmente. Beispiele für anorganische Absorptionspigmente sind Eisenoxidpigmente, Titandioxid und Ruß.

Farbstoffe sind ebenfalls Farbmittel und unterscheiden sich von den Pigmenten durch ihre Löslichkeit im Anwendungsmedium, d.h. sie weisen bei 25 °C eine Löslichkeit über 1 g / 1000 g im Anwendungsmedium auf.

Beispiele für Farbstoffe sind Azo-, Azin-, Anthrachinon-, Acridin-, Cyanin-, Oxazin-, Polymethin-, Thiazin-, Triarylmethan-Farbstoffe. Diese Farbstoffe können Anwendung finden als basische oder kationische Farbstoffe, Beizen-, Direkt-, Dispersions-, Entwicklungs-, Küpen-, Metallkomplex-, Reaktiv-, Säure-, Schwefel-, Kupplungs- oder Substantive Farbstoffe.

Als koloristisch inerte Füllstoffe sind alle Stoffe/Verbindungen zu verstehen, die einerseits koloristisch unwirksam sind; d.h. die eine geringe Eigenabsorption zeigen und deren Brechzahl ähnlich der Brechzahl des Beschichtungsmediums ist, und die andererseits in der Lage sind, die Orientierung (parallele Ausrichtung) der Effektpigmente in der Oberflächenbeschichtung, d.h. im applizierten Lackfilm, zu beeinflussen, ferner Eigenschaften der Beschichtung oder der Beschichtungsmassen, beispielsweise Härte oder Rheologie. Im Folgenden sind beispielhaft einsetzbare inerte Stoffe/Verbindungen genannt, ohne jedoch den Begriff koloristisch inerte topologiebeeinflussende Füllstoffe auf diese Beispiele zu beschränken. Geeignete inerte Füllstoffe entsprechend der Definition können beispielsweise transparente oder semitransparente Füllstoffe oder Pigmente sein, wie z.B. Kieselgele, Blancfixe, Kieselgur, Talkum, Calciumcarbonate, Kaolin, Bariumsulfat, Magnesiumsilikat, Aluminiumsilikat, kristallines Siliziumdioxid, amorphe Kieselsäure, Aluminiumoxid, Mikrokugeln oder Mikrohohlkugeln z.B. aus Glas, Keramik oder Polymeren mit Größen von beispielsweise 0,1-50 µm. Weiterhin können als inerte Füllstoffe beliebige feste inerte organische Partikel, wie z.B. Harnstoff-Formaldehyd-Kondensationsprodukte, mikronisiertes Polyolefinwachs und mikronisiertes Amidwachs, eingesetzt werden. Die inerten Füllstoffe können jeweils auch in Mischung eingesetzt werden. Bevorzugt wird jedoch jeweils nur ein Füllstoff eingesetzt.

Bevorzugte Füllstoffe umfassen Silikate, z. B. durch Hydrolyse von Siliciumtetrachlorid erhältliche Silikate wie Aerosil® der Fa. Degussa, Kieselerde, Talkum, Aluminiumsilikate, Magnesiumsilikate, Calciumcarbonate etc.

Nach Vermischen der Polyisocyanatkomponente mit einer Bindemittelkompontente und ggf. weiteren Komponenten wird das Lackgemisch bei Umgebungstemperatur bis 150 °C ausgehärtet.

In einer bevorzugten Variante wird das Lackgemisch bei Umgebungstemperatur bis 80 °C, besonders bevorzugt bis 60 °C ausgehärtet (z.B. für refinish-Anwendungen oder große Objekte, die sich schlecht in einen Ofen stellen lassen).

In einer anderen bevorzugten Anwendung wird das Lackgemisch bei 110-150 °C, bevorzugt bei 120-140 °C ausgehärtet (z.B. für OEM-Anwendungen).

Unter "Härtung" wird im Rahmen der vorliegenden Erfindung das Erzeugen einer klebfreien Beschichtung auf einem Substrat verstanden, indem man die auf das Substrat aufgetragene Beschichtungsmasse zumindest solange auf die oben angegebene Temperatur erwärmt, bis mindestens die gewünschte Klebfreiheit eingetreten ist.

In einer bevorzugten Form wird im Rahmen der vorliegenden Schrift unter einer Beschichtungsmasse eine Mischung zumindest zweier Komponenten (Bindemittel und Vernetzer) verstanden, die zum Beschichten mindestens eines Substrats zum Zwecke einer Ausbildung eines Films und, nach Härtung, einer klebfreien Beschichtung vorgesehen ist.

Die Beschichtung der Substrate erfolgt nach üblichen, dem Fachmann bekannten Verfahren, wobei man wenigstens eine Beschichtungsmasse auf das zu beschichtende Substrat in der gewünschten Stärke aufbringt und die optional enthaltenen flüchtigen Bestandteile der Beschichtungsmasse, optional unter Erhitzen, entfernt. Dieser Vorgang kann gewünschten falls ein- oder mehrfach wiederholt werden. Das Aufbringen auf das Substrat kann in bekannter Weise, z. B. durch Spritzen, Spachteln, Rakeln, Bürsten, Rollen, Walzen, Gießen, Laminieren, Hinterspritzen oder Koextrudieren erfolgen.

Die Dicke einer solchen zu härtenden Schicht kann von 0,1 µm bis mehrere mm betragen, bevorzugt von 1 bis 2.000 µm, besonders bevorzugt 5 bis 200 µm, ganz besonders bevorzugt von 5 bis 60 µm (bezogen auf den Lack im Zustand in dem das Lösungsmittel aus dem Lack entfernt ist).

Weiterhin sind auch Substrate, beschichtet mit einer erfindungsgemäßen Mehrschichtlackierung Gegenstand der vorliegenden Erfindung.

Besonders geeignet sind solche Polyurethanlacke für Anwendungen, in denen eine besonders hohe Applikationssicherheit, Außenwitterungsbeständigkeit, Optik, Lösemittel-, Chemikalien- und Wasserfestigkeit gefordert werden.

Die erhaltenen zweikomponentigen Beschichtungsmassen und Lackformulierungen eignen sich zum Beschichten von Substraten wie Holz, Holzfurnier, Papier, Pappe, Karton, Textil, Folie, Leder, Vlies, Kunststoffoberflächen, Glas, Keramik, mineralischen Baustoffen, wie Zement-Formsteine und Faserzementplatten oder Metallen, die jeweils optional vorbeschichtet bzw. vorbehandelt sein können.

Derartige Beschichtungsmassen eignen sich als oder in Innen- oder Außenbeschichtungen, also solche Anwendungen, die dem Tageslicht ausgesetzt sind, bevorzugt von Gebäudeteilen, Beschichtungen auf (Groß-)Fahrzeugen und Flugzeugen und industriellen Anwendungen, Nutzfahrzeuge im landwirtschaftlichen und Baubereich, Dekolackierungen, Brücken, Gebäuden, Strommasten, Tanks, Containern, Pipelines, Kraftwerken, chemischen Anlagen, Schiffen, Kränen, Pfählen, Spundwänden, Armaturen, Rohren, Fittings, Flanschen, Kupplungen, Hallen, Dächern und Baustahl, Möbeln, Fenstern, Türen, Parkett, Can-Coating und Coil-Coating, für Bodenbeläge, wie bei Parkdecks oder in Krankenhäusern, in Automobillacken als OEM und refinish-Anwendung.

Bevorzugt werden derartige Beschichtungsmassen bei Temperaturen zwischen Umgebungstemperatur bis 80 °C, bevorzugt bis 60 °C, besonders bevorzugt bis 40 °C eingesetzt. Bevorzugt handelt es sich dabei um solche Gegenstände, die nicht bei hohen Temperaturen gehärtet werden können, wie große Maschinen, Flugzeuge, Großraumfahrzeuge und refinish-Anwendungen.

Insbesondere werden die erfindungsgemäßen Beschichtungsmassen als Klar-, Basis- und Decklacke(n), Primern und Füllern eingesetzt.

Lange Lagerungen der Polyisocyanatkomponente sind insbesondere bei Refinish-, z.T. auch bei Industrieanwendungen üblich. Das mit antiflockulierend wirkenden Additiven versetzte Polyisocyanat respektive die Polyisocyanatkomponente kann natürlich auch für jede andere Anwendung eingesetzt werden.

### Beispiele

### Einsatzstoffe:

### Polyisocyanate

**PI-1 - PI-3, PI-5**: Verschiedene Chargen von Isocyanurat Basonat® HI 100 mit einer Viskosität von ca. 2800 mPa*s (BASF SE)
**PI-4**: Biuret Basonat® HB 100 (BASF SE)
**PI-6**: Isocyanurat von Isophorondiisocyanat, 70% in Solvesso® 100, Basonat® IT 270 S (BASF SE)
**PI-7**: Allophanat von Hexamethylendiisocyanat, Basonat® HA 100, Viskosität ca. 1200 mPa*s (BASF SE)
**PI-8**: Allophanat von Hexamethylendiisocyanat, Basonat® HA 300, Viskosität ca. 300 mPa*s (BASF SE)

**Additive**. Die in den folgenden Tabellen verwendeten Verbindungen werden wie folgt abgekürzt:
Sterisch gehinderte Phenole (Vergleich):
   - "BHT" = 2,6-Di-tert.-butyl-4-methyl-phenol
   - "1135" = Benzolpropansäure, 3,5-bis-(1,1-dimethyl-ethyl)-4-hydroxy-C7-C9 verzweigter Alkylester, Irganox® 1135 (BASF SE)
Standard-Wasserfänger (Vergleich):
   - "TI" = para-Toluolsulfonylisocyanat, Zusatzmittel TI (Borchers)
   - "OF" = Triethylorthoformat, Zusatzmittel OF (Borchers)
Einkernige Phosphite (Vergleich):
   - "Tppt" = Triphenylphosphit
   - "168" = Tris-(2, 4-di-tert-butylphenyl)-phosphat, Irgafos® 168 (BASF)
Säuren (Vergleich):
   - "Ac" = Essigsäure
   - "MS" = Methansulfonsäure
   - "TFAc" = Trifuoressigsäure
Sonstige Verbindungen (Vergleich):
   - "DPP" = Phosphonsäurediphenylester
Mehrkernige Phosphite (erfindungsgemäß):
   - "126" = Bis-(2,4-di-tert-butylphenyl)-pentaerythrol-diphosphit = Irgafos® 126 (BASF SE)
   - "9228" = 3,9-Bis(2,4-di-tert-butylphenoxy)-2,4,8,10-tetraoxa-3,9-diphosphaspiro[5.5]undecan = Pentaerythrol-diphosphit = Doverphos S-9228 (Dover Chemical Cooperation)
   - "618" = Weston 618 = Doverphos S 680 (CAS 3806-34-6)
   - 3,9-Bis(2,6-di-tert-butyl-4-methylphenoxy)-2,4,8,10-tetraoxa-3,9-diphosphaspiro(5.5)undecan (CAS 80693-00-1) = ADK Stab PEP-3
Phosphonite (erfindungsgemäß):
   - "EPQ" = Phosphortrichlorid, Reaktionsprodukte mit 1,1'- Biphenyl- und 2,4-bis(1,1-dimethylethyl)-phenol = Irgafos® P-EPQ (BASF SE)
Säuren
   - "2-CPS" = 2-Chlorpropionsäure
   - "3-CPS" = 3-Chlorpropionsäure
   - "DEHP" = Di-(ethylhexyl)-phosphat
   - "OPH" = Irgafos® OPH = Phosphonsäuredioctylester (BASF SE)
   - "DOPO" = Sanko HCA (CAS 35948-25-5) = 9,10-Dihydro-9-oxa-10-phosphaphenanthren-10-oxide (Sanko Co., Osaka, Japan)
   - "5076" = Dodecylbenzolsulfonsäure, Nacure® 5076 (King Industries)
   - "T-Ac" = Mercaptoessigsäure
   - "M-Ac" = Methoxyessigsäure

Zu den Lösungsmitteln wurden Wasser zugesetzt um eine schnellere Flockulation zu bewirken als mit den in käuflichen Lösungsmitteln vorliegenden Wassermengen. Die Gesamtmengen lagen bei 400-800 ppm Wasser gesamt bezogen auf Lösungsmitteln. Je höher die Wasserkonzentration, desto schneller erfolgt üblicherweise die Flockulation. Bei in der Praxis üblichen geringeren Wassermengen erfolgt Flockulation nach längeren Zeiträumen. Der Wassergehalt in den folgenden Versuchen bezieht sich auf die Lösungsmittel. Er wurde durch Karl-Fischer-Titration bestimmt.

Flockulationsmessung: Zur Bestimmung der Flockulation wurden üblicherweise 30 oder 40%-ige Lösungen von Polyisocyanat in Lösungsmitteln oder Lösungsmittelmischungen gelöst. Additive wurden über die Lösungsmittel eingebracht. 50 g der Mischung wurden in 50 mL Schraubdeckelgefäßen ohne seitliche Beschriftung mit Stickstoff überströmt und fest verschlossen bei 23 °C (50% Luftfeuchtigkeit) gelagert. Die Gefäße wurden üblicherweise in den ersten zwei Wochen täglich ohne die Wochenenden, danach wöchentlich, ab 10 Wochen zweiwöchentlich visuell gegen einen dunklen Hintergrund begutachtet.

### Benotung der Flockulation:

- 0: letzte Messtag vor Flockulation oder keine Flockulation während des ganzen Experiments;
- 1a: der erste Messtag mit einer ganz leichten Trübung oder mit dem bloßen Auge kaum sichtbaren feiner Bodensatz
- 1b: der letzte Messtag mit einer ganz leichten Trübung oder mit dem bloßen Auge kaum sichtbaren feiner Bodensatz
- 2: der erste Messtag mit einem deutlich erkennbaren Bodensatz oder Flockenbildung
- 3: angeliert
- X: keine Messung mehr

Die folgenden Beispiele sollen die Erfindung erläutern, aber nicht auf diese Beispiele beschränken.

Die Versuchsreihen zeigen, dass in den erfindungsgemäßen Beispielen längere Lagerzeiten ohne, respektive mit geringerer Flockulation erhalten werden als mit den Referenzbeispielen ohne Additive und nicht erfindungsgemäßen Additiven.
Zudem wird exemplarisch gezeigt, dass dieser Effekt in unterschiedlichen lacktypischen Lösungsmitteln wie n-Butylacetat (BuAc), Xylol/Butylacetat = 3:1, Xylol, Methoxypropylacetat MPA, Solvesso® 100 erhalten wird, wenn auch mit unterschiedlicher Ausprägung.
Zudem wurde der Effekt exemplarisch in Konzentrationen von 30 und 40% Polyisocyanat in Lösungsmitteln gezeigt, was lacktypisch ist, aber schon einer relativ starken Verdünnung entspricht.

**Versuchsreihe 1: Lagerung von PI-1 Isocyanurat 40% in n-Butylacetat mit einem Wassergehalt von ca. 800 ppm und verschiedenen Additiven mit Flockulierung / Niederschlagsbildung in Tagen über 126 Tage**

| Flockulation | Vgl. | Vgl. | Vgl. | Vgl. | Vgl. | Erf. | Erf. | Erf. | Erf. | Erf. | |
|---|---|---|---|---|---|---|---|---|---|---|---|
| Additiv | ohne | Tppt | 1135 | TI | TI | 126 | 9228 | EPQ | OPH | 1135 | EPQ |
| Menge (ppm) | 0 | 600 | 600 | 600 | 2000 | 600 | 600 | 200 | 600 | 200 | 600 |
| 0 (d) | 13 | 2 | 13 | 13 | 14 | 14 | 126 | 98 | 42 | 42 | |
| 1a (d) | 14 | 7 | 14 | 14 | 21 | 21 | X | 112 | 56 | 56 | |
| 1b (d) | 14 | 7 | 14 | 21 | 21 | 21 | X | 126 | 70 | 126 | |
| 2 (d) | 21 | 7 | 21 | 21 | 28 | 28 | X | X | 84 | X | |

**Versuchsreihe 2: Lagerung von PI-1 Isocyanurat 40%-ig in Xylol / n-Butylacetat = 3:1 mit einem Wassergehalt von ∼800 ppm und verschiedenen Additiven über 126 Tage**

| Flockulation | Vgl. | Vgl. | Erf. | Erf. | Erf. | | Erf. | |
|---|---|---|---|---|---|---|---|---|
| Additiv | ohne | Tppt | 9228 | EPQ | 1135 | EPQ | 1135 | OPH |
| Menge (ppm) | 0 | 600 | 600 | 600 | 200 | 600 | 200 | 600 |
| 0 (d) | 9 | 3 | 11 | 28 | 126 | | 98 | |
| 1a (d) | 10 | 8 | 14 | 42 | X | | 112 | |
| 1b (d) | 14 | 8 | 126 | 126 | X | | 126 | |
| 2 (d) | 21 | 8 | X | X | X | | X | |

**Versuchsreihe 3: Lagerung von PI-1 Isocyanurat 40% Xylol mit einem Wassergehalt von ∼400 ppm (gesättigte Lösung) und verschiedenen Additiven über 210 Tage**

| | Vgl. | Vgl. | Erf. | Erf. | Erf. | Erf. | | Erf. | |
|---|---|---|---|---|---|---|---|---|---|
| Additiv | ohne | Tppt | 9228 | EPQ | 126 | 1135 | EPQ | 1135 | OPH |
| Menge (ppm) | 0 | 600 | 600 | 600 | 600 | 200 | 600 | 200 | 600 |
| 0 (d) | 42 | 0 | 98 | 98 | 210 | 210 | | 210 | |
| 1a (d) | 56 | 5 | 112 | 112 | X | X | | X | |
| 1b (d) | 98 | 5 | 112 | 140 | X | X | | X | |
| 2 (d) | 112 | 5 | 112 | X | X | X | | X | |

**Versuchsreihe 4: Lagerung von PI-1 Isocyanurat 40 % in Solvesso® 100 mit einem Wassergehalt von ∼400 (gesättigte Lösung) und verschiedenen Additiven über 126 Tage**

| | Vgl. | Vgl. | Vgl. | Vgl. | Vgl. | Vgl. | Vgl. | Erf. | Erf. | Erf. | Erf. |
|---|---|---|---|---|---|---|---|---|---|---|---|
| Additiv | ohne | Tppt | 168 | TI | TI | OF | OF | 618 | EPQ | 126 | OPH |
| Menge (ppm) | 0 | 600 | 600 | 600 | 2000 | 600 | 2000 | 600 | 200 | 600 | 600 |
| 0 (d) | 14 | 0 | 14 | 84 | 84 | 84 | 84 | 126 | 126 | 126 | 126 |
| 1a (d) | 21 | 5 | 21 | 98 | 98 | 98 | 98 | X | X | X | X |
| 1b (d) | 126 | 5 | 28 | 98 | 98 | 98 | 98 | X | X | X | X |
| 2 (d) | X | 5 | 42 | 98 | 98 | 98 | 98 | X | X | X | X |

**Versuchsreihe 5: Lagerung von PI-2 Isocyanurat 40 % in Butylacetat mit einem Wassergehalt von -800 ppm und verschiedenen Additiven über 210 Tage**

| | Vgl. | Erf. | Erf. | Erf. |
|---|---|---|---|---|
| Additiv | ohne | DOPO | 126 | OPH |
| Menge (ppm) | 0 | 200 | 200 | 200 |
| 0 (d) | 5 | 6 | 7 | 5 |
| 1a (d) | 6 | 7* | 12* | 6* |
| 1b (d) | 12 | 210* | 210* | 210* |
| 2 (d) | 13 | X | X | X |

| | | | | |
|---|---|---|---|---|
| * Die Probe wies eine sehr leichte Trübung auf, die nach ca. 12 Wochen Lagerzeit nicht mehr zu erkennen war. Es wird angenommen, dass diese Werte im Grenzbereich der optischen Wahrnehmung liegen. Technische Hilfsmittel wurden nicht zur Verfügung genommen. | | | | |

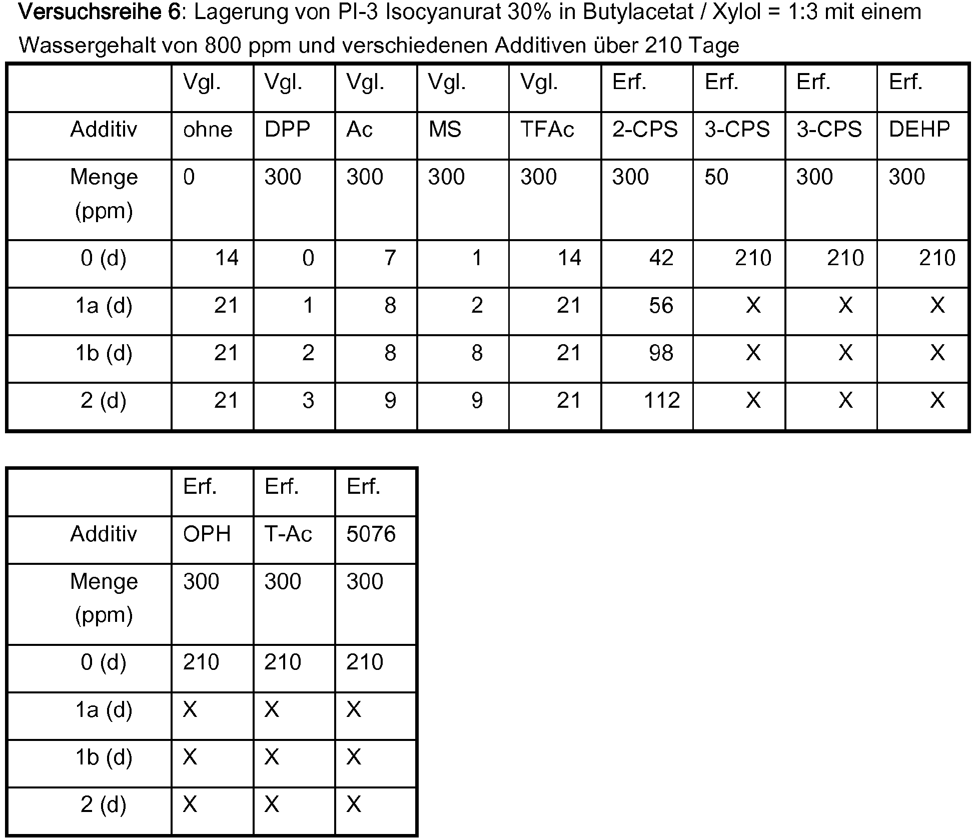

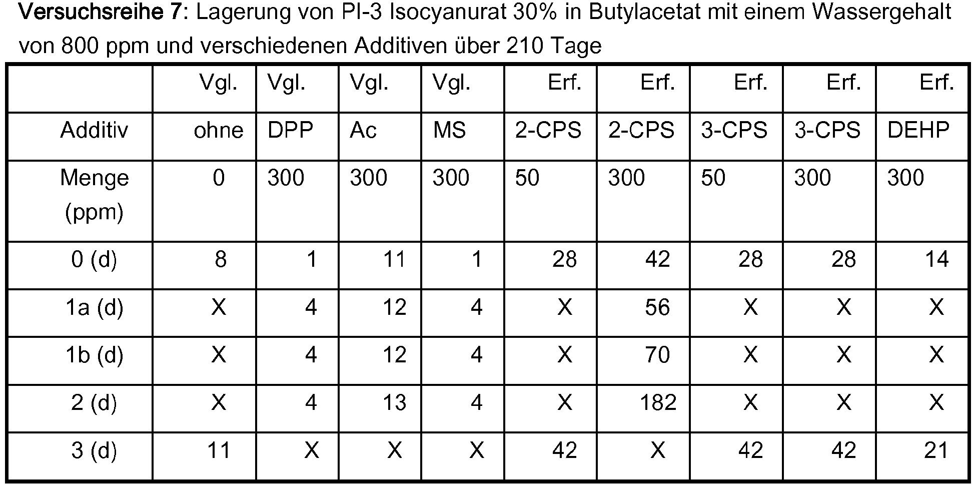

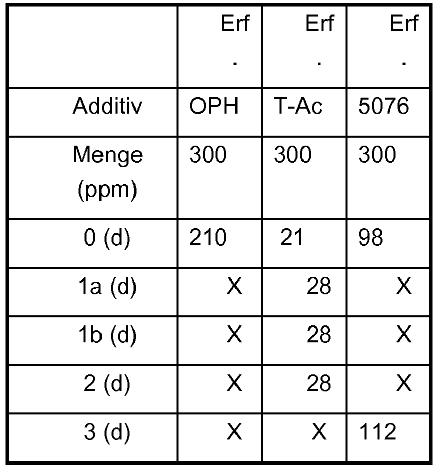

**Versuchsreihe 8: Lagerung von PI-4 Biuret 30% in Butylacetat mit einem Wassergehalt von 800 ppm und verschiedenen Additiven über 210 Tage**

| | Vgl. | Erf. | Erf. |
|---|---|---|---|
| Additiv | ohne | OPH | 126 |
| Menge (ppm) | 0 | 300 | 300 |
| 0 (d) | 8 | 112 | 98 |
| 1a (d) | 11 | 126 | X |
| 1b (d) | 12 | 182 | X |
| 2 (d) | 13 | 196 | X |
| 3 (d) | X | X | 182 |

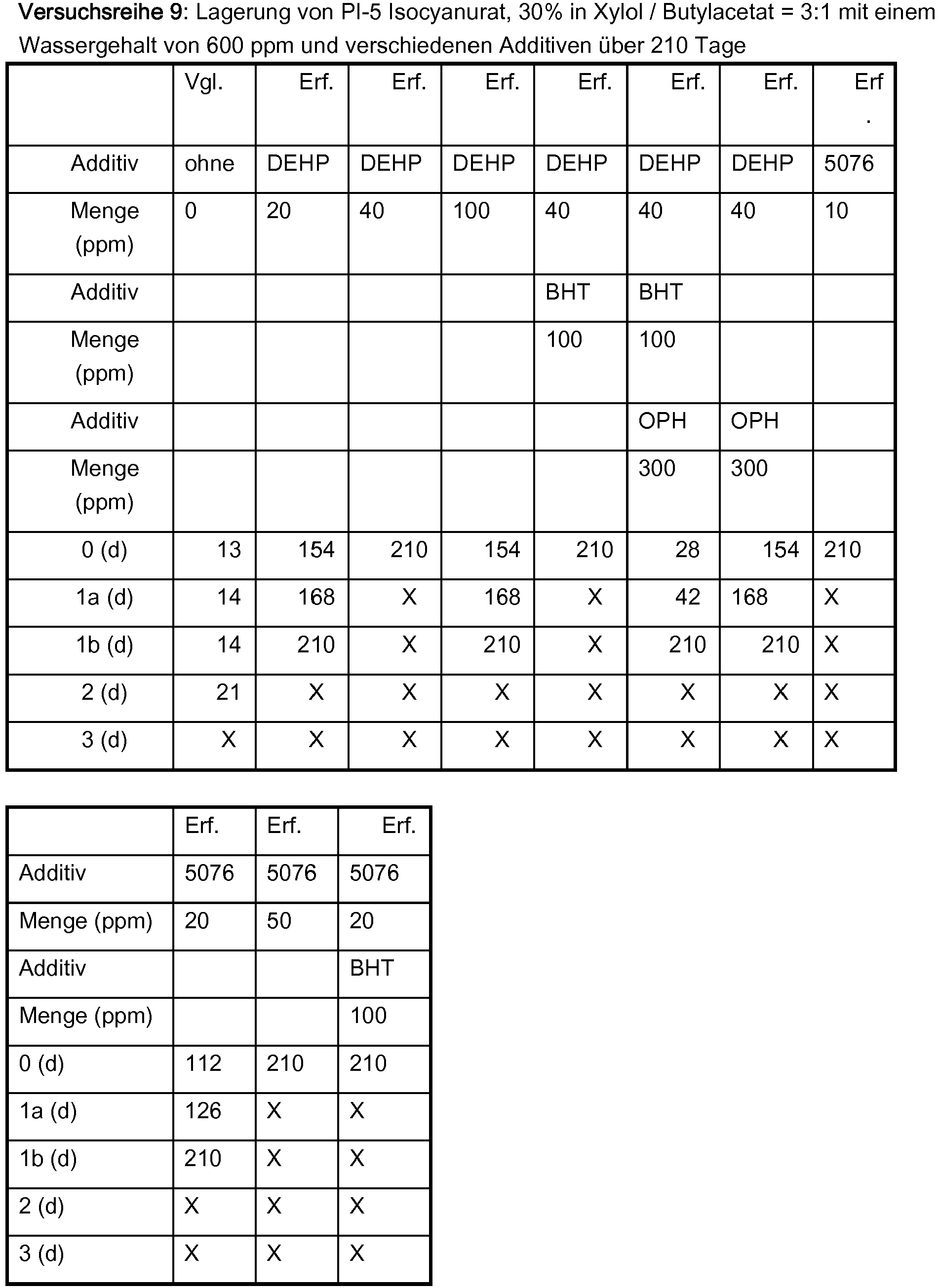

**Versuchsreihe 10: Lagerung von**

| a) PI-5 Basonat® HI 100 / PI-6Basonat® IT 270 S = 70:30 fest/fest, | | | | |
|---|---|---|---|---|
| b) PI-7 Basonat® HA 100 | | | | |
| c) PI-8 Basonat® HA 300 | | | | |
| 30% (Gesamtfeststoff) in Solvesso® 100 mit einem Wassergehalt von 100 ppm und Additiv | | | | |
| | Vgl. | Erf. | Vgl. | Erf. |
| Polyisocyanat | PI-7 | PI-7 | PI-8 | PI-8 |
| Additiv | ohne | DEHP | ohne | DEHP |
| Menge (ppm) | 0 | 50 | 0 | 50 |
| 0 (d) | 56 | 210 | 140 | 210 |
| 1a (d) | 70 | X | 154 | X |
| 1b (d) | 140 | X | 210 | X |
| 2 (d) | 154 | X | X | X |
| 3 (d) | X | X | X | X |

**Versuchsreihe 11: Wie Versuchsreihe 10, jedoch in Butylacetat mit einem Wassergehalt von 400 ppm und Additiv**

| | Vgl. | Erf. | Vgl. | Erf. |
|---|---|---|---|---|
| Polyisocyanat | PI-5/PI-6 | PI-5/PI-6 | PI-8 | PI-8 |
| Additiv | ohne | DEHP | ohne | DEHP |
| Menge (ppm) | 0 | 50 | 0 | 50 |
| 0 (d) | 56 | 182 | 126 | 168 |
| 1a (d) | 70 | 196 | 140 | 182 |
| 1b (d) | 70 | 210 | 210 | 210 |
| 2 (d) | 84 | X | X | X |
| 3 (d) | X | X | X | X |

**Versuchsreihe 12: Wie Versuchsreihe 10, jedoch Xylol / Butylacetat = 3:1 mit einem Wassergehalt von ca. 200 ppm und Additiv**

| | Vgl. | Erf. | Vgl. | Erf. | Vgl. | Erf. |
|---|---|---|---|---|---|---|
| Polyisocyanat | PI-5/PI-6 | PI-5/PI-6 | PI-7 | PI-7 | PI-8 | PI-8 |
| Additiv | ohne | DEHP | ohne | DEHP | ohne | DEHP |
| Menge (ppm) | 0 | 50 | 0 | 50 | 0 | 50 |
| 0 (d) | 56 | 182 | 42 | 182 | 70 | 210 |
| 1a (d) | 70 | 196 | 56 | 196 | 84 | X |
| 1b (d) | 70 | 210 | 70 | 210 | 210 | X |
| 2 (d) | 70 | X | 84 | X | X | X |
| 3 (d) | X | X | X | X | X | X |

## Patentansprüche

1. Verwendung von Additiven ausgewählt aus der Gruppe bestehend aus
a1) organischen Säuren mit einem pKs-Wert von unter 4,2, ausgewählt aus der Gruppe bestehend aus a1a) aromatischen Sulfonsäuren und a1b) ein- oder zweifach mit Alkoxy-, Mercapto- oder Alkylmercapto-substituierte zwei Kohlenstoffatome aufweisende Alkancarbonsäuren, ein- oder zweifach mit Halogen-, Alkoxy-, Mercapto- oder Alkylmercapto-substituierte, mindestens drei Kohlenstoffatome aufweisende Alkancarbonsäuren, Alkendicarbonsäuren oder Alkandicarbonsäuren
a2) Phosphiten der Formel 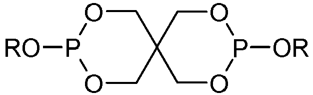 wobei R bevorzugt eine Aryl-Gruppe ist, die in 2, 4, und 6-Position wie folgt substituiert ist:
Position 2: tert. Butyl, tert. Amyl
Position 4: Wasserstoff, Alkyl, tert. Butyl oder tert. Amyl, und
Position 6: Wasserstoff, Alkyl, tert. Butyl oder tert. Amyl,
mit der Maßgabe, dass mindestens einer der Substituenten in Position 4 und 6 ungleich Wasserstoff ist,
a3) Phosphoniten der Formel (RO)₂P-X-P(RO)₂
wobei R bevorzugt eine Aryl-Gruppe ist, die in 2, 4, und 6-Position wie folgt substituiert ist:
Position 2: tert. Butyl, tert. Amyl
Position 4: Wasserstoff, Alkyl, tert. Butyl oder tert. Amyl, und
Position 6: Wasserstoff, Alkyl, tert. Butyl oder tert. Amyl,
mit der Maßgabe, dass mindestens einer der Substituenten in Position 4 und 6 ungleich Wasserstoff ist, und
X dabei eine Arylengruppe sei,
a4) sauren Phosphor-Derivaten, ausgewählt aus der Gruppe bestehend aus a4a) Mono- und Di-C₁- bis C₁₂-Alkylphosphaten, a4b) Mono- und Di-C₁- bis C₁₂-Alkylphosphonaten, a4c) Mono-C₁- bis C₁₂-Alkylphosphinaten und a4d) Alkylderivate von phosphorhaltigen Disäuren
zur Verringerung von Flockulation und/oder Niederschlagsbildung bei Exposition gegenüber (Luft-)Feuchtigkeit während der Lagerung von Polyisocyanatgemischen, welche mindestens ein Lösungsmittel enthalten.

2. Verwendung von Additiven nach Anspruch 1 **dadurch gekennzeichnet, dass** die Polyisocyanate (cyclo)aliphatisch sind.

3. Verwendung von Additiven nach Anspruch 1 **dadurch gekennzeichnet, dass** die Polyisocyanate auf Hexamethylendiisocyanat und/oder Isophorondiisocyanat als Monomeren basieren.

4. Verwendung von Additiven nach einem der vorstehenden Ansprüche, **dadurch gekennzeichnet, dass** die Polyisocyanate Isocyanurat-, Biuret oder Allophanat-(gegebenenfalls mit Urethan-)-Strukturen aufweisen.

5. Verwendung von Additiven nach einem der vorstehenden Ansprüche, **dadurch gekennzeichnet, dass** das Polyisocyanat Isocyanurat-, Urethan- und/oder Allophanatgruppen enthält, die unter Verwendung eines Ammoniumcarboxylat- oder Ammonium α-hydroxycarboxylat-Katalysators erhalten wurden.

6. Verwendung von Additiven nach einem der Ansprüche 1 bis 4, **dadurch gekennzeichnet, dass** es sich bei den Polyisocyanaten um Isocyanuratgruppen aufweisende Polyisocyanate handelt, die durch thermische Desaktivierung des Katalysators erhalten wurden.

7. Verwendung von Additiven nach einem der Ansprüche 1 bis 4, **dadurch gekennzeichnet, dass** es sich bei den Polyisocyanaten um Isocyanuratgruppen aufweisende Polyisocyanate handelt, die durch chemische Desaktivierung des Katalysators durch Zugabe von Deaktivatoren erhalten wurden.

8. Verwendung von Additiven nach Anspruch 1 **dadurch gekennzeichnet, dass** diese Additive unterstöchiometrisch in Bezug auf die im Polyisocyanatgemisch vorliegende Wassermenge eingesetzt werden.

9. Verwendung von Additiven nach Anspruch 1 **dadurch gekennzeichnet, dass** die Additive ausgewählt sind aus der Gruppe bestehend aus 2-Chlorpropionsäure, 3-Chlorpropionsäure, Di-(2-ethylhexyl)-phosphat, Dibutylphosphat, 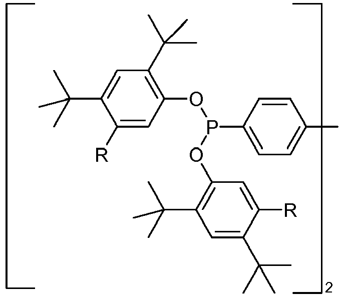 (CAS 26741-53-7) mit R = H und Methyl, Bis-(2,4-di-tert-butylphenyl)-pentaerythrol-diphosphit (CAS 26741-53-7), Dioctylphosphonsäureester, Di-(2-ethylhexyl)-phosphonsäureester, Di-n-butylphosphonsäureester, Diethylphosphonsäureester, Dimethylphosphonsäureester, 9,10-Dihydro-9-oxa-10-phosphaphenanthren-10-oxide (CAS 35948-25-5) und Dodecylbenzolsulfonsäure.

10. Verwendung von Additiven nach Anspruch 1 **dadurch gekennzeichnet, dass** die mit Additiv versetzten Polyisocyanate zusätzlich mindestens ein Phenol oder überbrücktes Bisphenol enthalten, das an dem aromatischen Ring je genau eine phenolische Hydroxygruppe aufweist und in den ortho-Positionen zur phenolischen Hydroxygruppe Alkylgruppen aufweist.

11. Verwendung von Additiven nach Anspruch 10 **dadurch gekennzeichnet, dass** die mit Additiv versetzten Polyisocyanate zusätzlich mindestens ein Dialkylphosphat, Dialkylphosphonat und/oder eine aromatische Sulfonsäure enthalten.

12. Verwendung von Additiven nach Anspruch 10 und 11 **dadurch gekennzeichnet, dass** die mit Additiv versetzten Polyisocyanate zusätzlich mindestens eine Lewis-Säure enthalten.

13. Verfahren zur Stabilisierung von Polyisocyanaten und Polyisocyanatgemischen in Lösungsmittel gegen Flockulation während der Lagerung, **dadurch gekennzeichnet, dass** man mindestens ein Additiv ausgewählt aus der Gruppe bestehend aus
a1) organischen Säuren mit einem pKs-Wert von unter 4,2, ausgewählt aus der Gruppe bestehend aus a1a) aromatischen Sulfonsäuren und a1b) ein- oder zweifach mit Alkoxy-, Mercapto- oder Alkylmercapto-substituierte zwei Kohlenstoffatome aufweisende Alkancarbonsäuren, ein- oder zweifach mit Halogen-, Alkoxy-, Mercapto- oder Alkylmercapto-substituierte, mindestens drei Kohlenstoffatome aufweisende Alkancarbonsäuren, Alkendicarbonsäuren oder Alkandicarbonsäuren
a2) Phosphiten der Formel 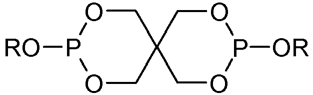 wobei R bevorzugt eine Aryl-Gruppe ist, die in 2, 4, und 6-Position wie folgt substituiert ist:
Position 2: tert. Butyl, tert. Amyl
Position 4: Wasserstoff, Alkyl, tert. Butyl oder tert. Amyl, und
Position 6: Wasserstoff, Alkyl, tert. Butyl oder tert. Amyl,
mit der Maßgabe, dass mindestens einer der Substituenten in Position 4 und 6 ungleich Wasserstoff ist,
a3) Phosphoniten der Formel (RO)₂P-X-P(RO)₂
wobei R bevorzugt eine Aryl-Gruppe ist, die in 2, 4, und 6-Position wie folgt substituiert ist:
Position 2: tert. Butyl, tert. Amyl
Position 4: Wasserstoff, Alkyl, tert. Butyl oder tert. Amyl, und
Position 6: Wasserstoff, Alkyl, tert. Butyl oder tert. Amyl,
mit der Maßgabe, dass mindestens einer der Substituenten in Position 4 und 6 ungleich Wasserstoff ist, und
X dabei eine Arylengruppe sei,
a4) sauren Phosphor-Derivaten, ausgewählt aus der Gruppe bestehend aus a4a) Mono- und Di-C₁- bis C₁₂-Alkylphosphaten, a4b) Mono- und Di-C₁- bis C₁₂-Alkylphosphonaten, a4c) Mono-C₁- bis C₁₂-Alkylphosphinaten und a4d) Alkylderivate von phosphorhaltigen Disäuren
zum Polyisocyanat oder Polyisocyanatgemisch gibt.

14. Verfahren zur Stabilisierung von Polyisocyanaten und Polyisocyanatgemischen gegen Flockulation nach Anspruch 13, **dadurch gekennzeichnet, dass** die Mischungen Lewis-Säuren wie beispielsweise Zinn-, Zink, Bismut-, Titan, Zirkonium-organische Verbindungen als Katalysatoren und/oder Antioxidantien und/oder andere übliche Lackadditive enthalten.

15. Verfahren zur Beschichtung von Substraten mit, gegen Flockulation während der Lagerung in Lösungsmitteln stabilisierten Polyisocyanaten und Polyisocyanatgemischen, **dadurch gekennzeichnet, dass** man mindestens ein Additiv ausgewählt aus der Gruppe bestehend aus
a1) organischen Säuren mit einem pKs-Wert von unter 4,2, ausgewählt aus der Gruppe bestehend aus a1a) aromatischen Sulfonsäuren und a1b) ein- oder zweifach mit Alkoxy-, Mercapto- oder Alkylmercapto-substituierte zwei Kohlenstoffatome aufweisende Alkancarbonsäuren, ein- oder zweifach mit Halogen-, Alkoxy-, Mercapto- oder Alkylmercapto-substituierte, mindestens drei Kohlenstoffatome aufweisende Alkancarbonsäuren, Alkendicarbonsäuren oder Alkandicarbonsäuren
a2) Phosphiten der Formel 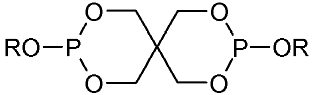 wobei R bevorzugt eine Aryl-Gruppe ist, die in 2, 4, und 6-Position wie folgt substituiert ist:
Position 2: tert. Butyl, tert. Amyl
Position 4: Wasserstoff, Alkyl, tert. Butyl oder tert. Amyl, und
Position 6: Wasserstoff, Alkyl, tert. Butyl oder tert. Amyl,
mit der Maßgabe, dass mindestens einer der Substituenten in Position 4 und 6 ungleich Wasserstoff ist,
a3) Phosphoniten der Formel (RO)₂P-X-P(RO)₂
wobei R bevorzugt eine Aryl-Gruppe ist, die in 2, 4, und 6-Position wie folgt substituiert ist:
Position 2: tert. Butyl, tert. Amyl
Position 4: Wasserstoff, Alkyl, tert. Butyl oder tert. Amyl, und
Position 6: Wasserstoff, Alkyl, tert. Butyl oder tert. Amyl,
mit der Maßgabe, dass mindestens einer der Substituenten in Position 4 und 6 ungleich Wasserstoff ist, und
X dabei eine Arylengruppe sei,
a4) sauren Phosphor-Derivaten, ausgewählt aus der Gruppe bestehend aus a4a) Mono- und Di-C₁- bis C₁₂-Alkylphosphaten, a4b) Mono- und Di-C₁- bis C₁₂-Alkylphosphonaten, a4c) Mono-C₁ bis C₁₂-Alkylphosphinaten und a4d) Alkylderivate von phosphorhaltigen Disäuren
zum Polyisocyanat oder Polyisocyanatgemisch zusetzt und dieses mit einem Lösungsmittel und optional anderen Additiven versetzt, lagert, und in einem Folgeschritt mit mindestens einer Bindemittel enthaltenden Komponente versetzt und anschließend auf ein Substrat aufbringt.

16. Verfahren gemäß Anspruch 15, **dadurch gekennzeichnet, dass** das die Bindemittel enthaltende Komponenten Verbindungen ausgewählt aus der Gruppe bestehend aus Polyacrylatpolyolen, Polyesterpolyolen, Polyurethanpolyolen, Polycarbonatpolyolen und Polyetherpolyolen enthalten.

17. Verwendung von Polyisocyanatkomponenten erhalten nach einem der Ansprüche 15 oder 15 in Beschichtungsmitteln in Grundierungen, Füllern, pigmentierten Decklacken, Basislacken und Klarlacken im Bereich Autoreparatur- oder Großfahrzeuglackierung sowie bei Nutzfahrzeugen im landwirtschaftlichen und Baubereich.

## Claims

1. The use of additives selected from the group consisting of
a1) organic acids having a pKa of below 4.2, selected from the group consisting of a1a) aromatic sulfonic acids and a1b) singularly or doubly alkoxy-, mercapto- or alkylmercapto-substituted alkanecarboxylic acids having two carbon atoms, singularly or doubly halogen-, alkoxy-, mercapto- or alkylmercapto-substituted alkanecarboxylic acids, alkenedicarboxylic acids or alkanedicarboxylic acids having at least three carbon atoms,
a2) phosphites of the formula 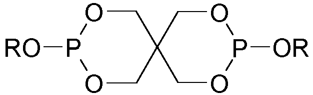 where R is preferably an aryl group which is substituted in positions 2, 4 and 6 as follows:
Position 2: tert-butyl, tert-amyl,
Position 4: hydrogen, alkyl, tert-butyl or tert-amyl, and
Position 6: hydrogen, alkyl, tert-butyl or tert-amyl,
with the proviso that at least one of the substituents in positions 4 and 6 is not hydrogen,
a3) phosphonites of the formula (RO)₂P-X-P(RO)₂ where R is preferably an aryl group which is substituted in positions 2, 4 and 6 as follows:
Position 2: tert-butyl, tert-amyl,
Position 4: hydrogen, alkyl, tert-butyl or tert-amyl, and
Position 6: hydrogen, alkyl, tert-butyl or tert-amyl,
with the proviso that at least one of the substituents in positions 4 and 6 is not hydrogen, and
X in this case is an arylene group,
a4) acidic phosphorus derivatives selected from the group consisting of a4a) mono- and di-C₁ to C₁₂ alkyl phosphates, a4b) mono- and di-C₁ to C₁₂ alkyl phosphonates, a4c) mono-C₁ to C₁₂ alkyl phosphinates, and a4d) alkyl derivatives of phosphorus-containing diacids,
for reducing flocculation and/or precipitation on exposure to (atmospheric) moisture during storage of polyisocyanate mixtures which comprise at least one solvent.

2. The use of additives according to claim 1, wherein the polyisocyanates are (cyclo)aliphatic.

3. The use of additives according to claim 1, wherein the polyisocyanates are based on hexamethylene diisocyanate and/or isophorone diisocyanate as monomers.

4. The use of additives according to any of the preceding claims, wherein the polyisocyanates have isocyanurate, biuret or allophanate (optionally with urethane) structures.

5. The use of additives according to any of the preceding claims, wherein the polyisocyanate comprises isocyanurate, urethane and/or allophanate groups obtained using an ammonium carboxylate or ammonium α -hydroxycarboxylate catalyst.

6. The use of additives according to any of claims 1 to 4, wherein the polyisocyanates are polyisocyanates which contain isocyanurate groups and have been obtained by thermal deactivation of the catalyst.

7. The use of additives according to any of claims 1 to 4, wherein the polyisocyanates are polyisocyanates which contain isocyanurate groups and have been obtained by chemical deactivation of the catalyst by addition of deactivators.

8. The use of additives according to claim 1, wherein these additives are used substoichiometrically in relation to the amount of water present in the polyisocyanate mixture.

9. The use of additives according to claim 1, wherein the additives are selected from the group consisting of 2-chloropropionic acid, 3-chloropropionoic acid, di(2-ethylhexyl) phosphate, dibutyl phosphate, 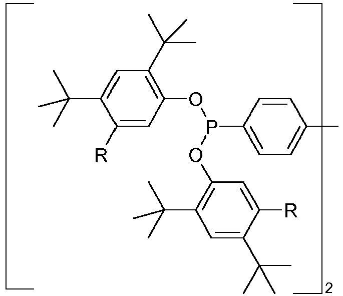 (CAS 26741-53-7) with R = H and methyl, bis(2,4-di-tert-butylphenyl) pentaerythritol diphosphite (CAS 26741-53-7), dioctylphosphonic esters, di(2-ethylhexyl)phosphonic esters, di-n-butylphosphonic esters, diethylphosphonic esters, dimethylphosphonic esters, 9,10-dihydro-9-oxa-10-phosphaphenanthrene 10-oxides (CAS 35948-25-5), and dodecylbenzenesulfonic acid.

10. The use of additives according to claim 1, wherein the polyisocyanates admixed with additive further comprise at least one phenol or bridged bisphenol which has exactly one phenolic hydroxyl group on the aromatic ring and has alkyl groups in the positions ortho to the phenolic hydroxyl group.

11. The use of additives according to claim 10, wherein the polyisocyanates admixed with additive further comprise at least one dialkyl phosphate, dialkyl phosphonate and/or an aromatic sulfonic acid.

12. The use of additives according to claim 10 and 11, wherein the polyisocyanates admixed with additive further comprise at least one Lewis acid.

13. A method for stabilizing polyisocyanates and polyisocyanate mixtures in solvent against flocculation during storage, wherein the polyisocyanate or polyisocyanate mixture is admixed with at least one additive selected from the group consisting of
a1) organic acids having a pKa of below 4.2, selected from the group consisting of a1a) aromatic sulfonic acids and a1b) singularly or doubly alkoxy-, mercapto- or alkylmercapto-substituted alkanecarboxylic acids having two carbon atoms, singularly or doubly halogen-, alkoxy-, mercapto- or alkylmercapto-substituted alkanecarboxylic acids, alkenedicarboxylic acids or alkanedicarboxylic acids having at least three carbon atoms,
a2) phosphites of the formula 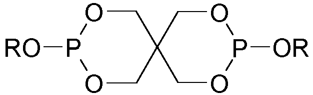 where R is preferably an aryl group which is substituted in positions 2, 4 and 6 as follows:
Position 2: tert-butyl, tert-amyl,
Position 4: hydrogen, alkyl, tert-butyl or tert-amyl, and
Position 6: hydrogen, alkyl, tert-butyl or tert-amyl,
with the proviso that at least one of the substituents in positions 4 and 6 is not hydrogen,
a3) phosphonites of the formula (RO)₂P-X-P(RO)₂
where R is preferably an aryl group which is substituted in positions 2, 4 and 6 as follows:
Position 2: tert-butyl, tert-amyl,
Position 4: hydrogen, alkyl, tert-butyl or tert-amyl, and
Position 6: hydrogen, alkyl, tert-butyl or tert-amyl,
with the proviso that at least one of the substituents in positions 4 and 6 is not hydrogen, and
X in this case is an arylene group,
a4) acidic phosphorus derivatives selected from the group consisting of a4a) mono- and di-C₁ to C₁₂ alkyl phosphates, a4b) mono- and di-C₁ to C₁₂ alkyl phosphonates, a4c) mono-C₁ to C₁₂ alkyl phosphinates, and a4d) alkyl derivatives of phosphorus-containing diacids.

14. The method for stabilizing polyisocyanates and polyisocyanate mixtures against flocculation according to claim 13, wherein the mixtures comprise Lewis acids such as, for example, organotin, organozinc, organobismuth, organotitanium, and organozirconium compounds as catalysts and/or antioxidants and/or other customary coatings additives.

15. A method for coating substrates with polyisocyanates and polyisocyanate mixtures stabilized against flocculation during storage in solvents, wherein the polyisocyanate or polyisocyanate mixture is admixed with at least one additive selected from the group consisting of
a1) organic acids having a pKa of below 4.2, selected from the group consisting of a1a) aromatic sulfonic acids and a1b) singularly or doubly alkoxy-, mercapto- or alkylmercapto-substituted alkanecarboxylic acids having two carbon atoms, singularly or doubly halogen-, alkoxy-, mercapto- or alkylmercapto-substituted alkanecarboxylic acids, alkenedicarboxylic acids or alkanedicarboxylic acids having at least three carbon atoms,
a2) phosphites of the formula 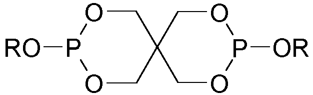 where R is preferably an aryl group which is substituted in positions 2, 4 and 6 as follows:
Position 2: tert-butyl, tert-amyl,
Position 4: hydrogen, alkyl, tert-butyl or tert-amyl, and
Position 6: hydrogen, alkyl, tert-butyl or tert-amyl,
with the proviso that at least one of the substituents in positions 4 and 6 is not hydrogen,
a3) phosphonites of the formula (RO)₂P-X-P(RO)₂
where R is preferably an aryl group which is substituted in positions 2, 4 and 6 as follows:
Position 2: tert-butyl, tert-amyl,
Position 4: hydrogen, alkyl, tert-butyl or tert-amyl, and
Position 6: hydrogen, alkyl, tert-butyl or tert-amyl, with the proviso that at least one of the substituents in positions 4 and 6 is not hydrogen, and
X in this case is an arylene group,
a4) acidic phosphorus derivatives selected from the group consisting of a4a) mono- and di-C₁ to C₁₂ alkyl phosphates, a4b) mono- and di-C₁ to C₁₂ alkyl phosphonates, a4c) mono-C₁ to C₁₂ alkyl phosphinates, and a4d) alkyl derivatives of phosphorus-containing diacids,
and this mixture is admixed with a solvent and optionally other additives, is stored, and in a subsequent step is admixed with at least one binder-comprising component, and is subsequently applied to a substrate.

16. The method according to claim 15, wherein the components comprising the binders comprise compounds selected from the group consisting of polyacrylate polyols, polyester polyols, polyurethane polyols, polycarbonate polyols, and polyether polyols.

17. The use of polyisocyanate components obtained according to either of claims 15 and 15 in coating compositions in primers, fillers, pigmented topcoats, basecoats and clearcoats in the automotive refinishing or large-vehicle finishing sector and also for utility vehicles in the agricultural and construction sectors.

## Revendications

1. Utilisation d'additifs choisis dans le groupe constitué par :
a1) les acides organiques ayant une valeur de pKs inférieure à 4,2, choisis dans le groupe constitué par a1a) les acides sulfoniques aromatiques et a1b) les acides alcanecarboxyliques comprenant deux atomes de carbone, substitués une ou deux fois avec alcoxy, mercapto ou alkylmercapto, les acides alcanecarboxyliques comprenant au moins trois atomes de carbone, substitués une ou deux fois avec halogène, alcoxy, mercapto ou alkylmercapto, les acides alcènedicarboxyliques ou les acides alcanedicarboxyliques,
a2) les phosphites de formule 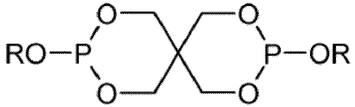 dans laquelle R représente de préférence un groupe aryle, qui est substitué de la manière suivante en position 2, 4 et 6 :
position 2 : tert.-butyle, tert.-amyle,
position 4 : hydrogène, alkyle, tert.-butyle ou tert.-amyle, et
position 6 : hydrogène, alkyle, tert.-butyle ou tert.-amyle,
à condition qu'au moins un des substituants en position 4 et 6 soit différent de l'hydrogène,
a3) les phosphonites de formule (RO)₂P-X-P(RO)₂ dans laquelle R représente de préférence un groupe aryle, qui est substitué de la manière suivante en position 2, 4 et 6 :
position 2 : tert.-butyle, tert.-amyle,
position 4 : hydrogène, alkyle, tert.-butyle ou tert.-amyle, et
position 6 : hydrogène, alkyle, tert.-butyle ou tert.-amyle,
à condition qu'au moins un des substituants en position 4 et 6 soit différent de l'hydrogène, et
X représente un groupe arylène,
a4) les dérivés de phosphore acides, choisis dans le groupe constitué par a4a) les phosphates de mono- et dialkyle en C₁ à C₁₂, a4b) les phosphonates de mono- et dialkyle en C₁ à C₁₂, a4c) les phosphinates de mono-alkyle en C₁ à C₁₂, et a4d) les dérivés d'alkyle de diacides contenant du phosphore,
pour la réduction de la floculation et/ou de la formation de précipités lors de l'exposition à l'humidité (de l'air) pendant le stockage de mélanges de polyisocyanates qui contiennent au moins un solvant.

2. Utilisation d'additifs selon la revendication 1, **caractérisée en ce que** les polyisocyanates sont (cyclo)aliphatiques.

3. Utilisation d'additifs selon la revendication 1, **caractérisée en ce que** les polyisocyanates sont à base de diisocyanate d'hexaméthylène et/ou de diisocyanate d'isophorone en tant que monomères.

4. Utilisation d'additifs selon l'une quelconque des revendications précédentes, **caractérisée en ce que** les polyisocyanates comprennent des structures isocyanurate, biuret ou allophanate (éventuellement avec des structures uréthane).

5. Utilisation d'additifs selon l'une quelconque des revendications précédentes, **caractérisée en ce que** le polyisocyanate contient des groupes isocyanurate, uréthane et/ou allophanate, qui ont été obtenus en utilisant un catalyseur à base de carboxylate d'ammonium ou d'α-hydroxycarboxylate d'ammonium.

6. Utilisation d'additifs selon l'une quelconque des revendications 1 à 4, **caractérisée en ce que** les polyisocyanates consistent en des polyisocyanates comprenant des groupes isocyanurate, qui ont été obtenus par désactivation thermique du catalyseur.

7. Utilisation d'additifs selon l'une quelconque des revendications 1 à 4, **caractérisée en ce que** les polyisocyanates consistent en des polyisocyanates comprenant des groupes isocyanurate, qui ont été obtenus par désactivation chimique du catalyseur par ajout de désactivateurs.

8. Utilisation d'additifs selon la revendication 1, **caractérisée en ce que** ces additifs sont utilisés en quantité sous-stoechiométrique par rapport à la quantité d'eau présente dans le mélange de polyisocyanates.

9. Utilisation d'additifs selon la revendication 1, **caractérisée en ce que** les additifs sont choisis dans le groupe constitué par l'acide 2-chloropropionique, l'acide 3-chloropropionique, le phosphate de di-(2-éthylhexyle), le phosphate de dibutyle, 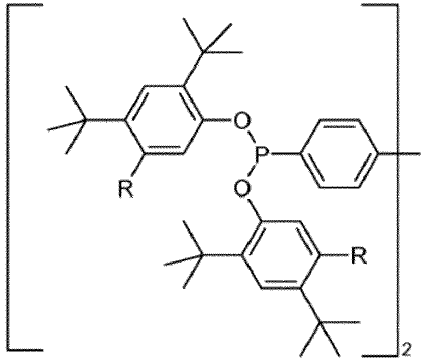 (CAS 26741-53-7) avec R = H et méthyle, le diphosphite de bis-(2,4-di-tert-butylphényl)-pentaérythrol (CAS 26741-53-7), les esters de l'acide dioctylphosphonique, les esters de l'acide di-(2-éthylhexyl)-phosphonique, les esters de l'acide di-n-butylphosphonique, les esters de l'acide diéthylphosphonique, les esters de l'acide diméthylphosphonique, le 9,10-dihydro-9-oxa-10-phosphaphénanthrène-10-oxyde (CAS 35948-25-5) et l'acide dodécylbenzène-sulfonique.

10. Utilisation d'additifs selon la revendication 1, **caractérisée en ce que** les polyisocyanates mélangés avec les additifs contiennent en outre au moins un phénol ou un bisphénol ponté, qui comprend à chaque fois exactement un groupe hydroxy phénolique sur le cycle aromatique et comprend des groupes alkyle aux positions ortho par rapport au groupe hydroxy phénolique.

11. Utilisation d'additifs selon la revendication 10, **caractérisée en ce que** les polyisocyanates mélangés avec les additifs contiennent en outre au moins un phosphate de dialkyle, un phosphonate de dialkyle et/ou un acide sulfonique aromatique.

12. Utilisation d'additifs selon les revendications 10 et 11, **caractérisée en ce que** les polyisocyanates mélangés avec les additifs contiennent en outre au moins un acide de Lewis.

13. Procédé de stabilisation de polyisocyanates et de mélanges de polyisocyanates dans des solvants contre la floculation pendant le stockage, **caractérisé en ce qu'**au moins un additif choisi dans le groupe constitué par :
a1) les acides organiques ayant une valeur de pKs inférieure à 4,2, choisis dans le groupe constitué par a1a) les acides sulfoniques aromatiques et a1b) les acides alcanecarboxyliques comprenant deux atomes de carbone, substitués une ou deux fois avec alcoxy, mercapto ou alkylmercapto, les acides alcanecarboxyliques comprenant au moins trois atomes de carbone, substitués une ou deux fois avec halogène, alcoxy, mercapto ou alkylmercapto, les acides alcènedicarboxyliques ou les acides alcanedicarboxyliques,
a2) les phosphites de formule 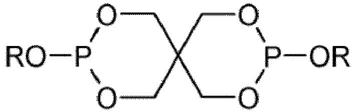 dans laquelle R représente de préférence un groupe aryle, qui est substitué de la manière suivante en position 2, 4 et 6 :
position 2 : tert.-butyle, tert.-amyle,
position 4 : hydrogène, alkyle, tert.-butyle ou tert.-amyle, et
position 6 : hydrogène, alkyle, tert.-butyle ou tert.-amyle,
à condition qu'au moins un des substituants en position 4 et 6 soit différent de l'hydrogène,
a3) les phosphonites de formule (RO)₂P-X-P(RO)₂ dans laquelle R représente de préférence un groupe aryle, qui est substitué de la manière suivante en position 2, 4 et 6 :
position 2 : tert.-butyle, tert.-amyle,
position 4 : hydrogène, alkyle, tert.-butyle ou tert.-amyle, et
position 6 : hydrogène, alkyle, tert.-butyle ou tert.-amyle,
à condition qu'au moins un des substituants en position 4 et 6 soit différent de l'hydrogène, et
X représente un groupe arylène,
a4) des dérivés de phosphore acides, choisis dans le groupe constitué par a4a) les phosphates de mono- et dialkyle en C₁ à C₁₂, a4b) les phosphonates de mono- et dialkyle en C₁ à C₁₂, a4c) les phosphinates de mono-alkyle en C₁ à C₁₂, et a4d) les dérivés d'alkyle de diacides contenant du phosphore,
est ajouté au polyisocyanate ou au mélange de polyisocyanates.

14. Procédé de stabilisation de polyisocyanates et de mélanges de polyisocyanates contre la floculation selon la revendication 13, **caractérisé en ce que** les mélanges contiennent des acides de Lewis tels que par exemple des composés organiques d'étain, de zinc, de bismuth, de titane, de zirconium en tant que catalyseurs et/ou des antioxydants et/ou d'autres additifs usuels pour les vernis.

15. Procédé de revêtement de substrats avec des polyisocyanates et des mélanges de polyisocyanates stabilisés contre la floculation pendant le stockage dans des solvants, **caractérisé en ce qu'**au moins un additif choisi dans le groupe constitué par :
a1) les acides organiques ayant une valeur de pKs inférieure à 4,2, choisis dans le groupe constitué par a1a) les acides sulfoniques aromatiques et a1b) les acides alcanecarboxyliques comprenant deux atomes de carbone, substitués une ou deux fois avec alcoxy, mercapto ou alkylmercapto, les acides alcanecarboxyliques comprenant au moins trois atomes de carbone, substitués une ou deux fois avec halogène, alcoxy, mercapto ou alkylmercapto, les acides alcènedicarboxyliques ou les acides alcanedicarboxyliques,
a2) les phosphites de formule 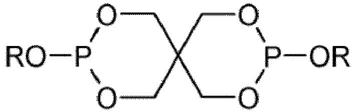 dans laquelle R représente de préférence un groupe aryle, qui est substitué de la manière suivante en position 2, 4 et 6 :
position 2 : tert.-butyle, tert.-amyle,
position 4 : hydrogène, alkyle, tert.-butyle ou tert.-amyle, et
position 6 : hydrogène, alkyle, tert.-butyle ou tert.-amyle,
à condition qu'au moins un des substituants en position 4 et 6 soit différent de l'hydrogène,
a3) les phosphonites de formule (RO)₂P-X-P(RO)₂ dans laquelle R représente de préférence un groupe aryle, qui est substitué de la manière suivante en position 2, 4 et 6 :
position 2 : tert.-butyle, tert.-amyle,
position 4 : hydrogène, alkyle, tert.-butyle ou tert.-amyle, et
position 6 : hydrogène, alkyle, tert.-butyle ou tert.-amyle,
à condition qu'au moins un des substituants en position 4 et 6 soit différent de l'hydrogène, et
X représente un groupe arylène,
a4) des dérivés de phosphore acides, choisis dans le groupe constitué par a4a) les phosphates de mono- et dialkyle en C₁ à C₁₂, a4b) les phosphonates de mono- et dialkyle en C₁ à C₁₂, a4c) les phosphinates de mono-alkyle en C₁ à C₁₂, et a4d) les dérivés d'alkyle de diacides contenant du phosphore,
est ajouté au polyisocyanate ou au mélange de polyisocyanates, et celui-ci est mélangé avec un solvant et éventuellement d'autres additifs, stocké et, lors d'une étape ultérieure, mélangé avec au moins un composant contenant un liant, puis appliqué sur un substrat.

16. Procédé selon la revendication 15, **caractérisé en ce que** les composants contenant le liant contiennent des composés choisis dans le groupe constitué par les polyacrylate-polyols, les polyester-polyols, les polyuréthane-polyols, les polycarbonate-polyols et les polyéther-polyols.

17. Utilisation de composants polyisocyanates obtenus selon l'une quelconque des revendications 15 ou 15 dans des agents de revêtement dans des apprêts primaires, des couches de remplissage, des vernis de recouvrement pigmentés, des vernis de base et des vernis transparents dans le domaine du vernissage de réparation automobile ou de véhicules de grandes dimensions, ainsi que pour des véhicules utilitaires dans le domaine de l'agriculture et de la construction.
